# EUROPEAN PATENT APPLICATION

(11) **EP 4 331 641 A1**
(43) Date of publication of application: **06.03.2024**
(21) Application number: 22795634.9
(22) Date of filing: 19.04.2022
(51) Int. Cl.: A61M 5/14, A61M 25/092

(54) **CATHETER DEVICE AND DRUG SOLUTION INJECTION DEVICE**

(30) Priority: 30.04.2021 JP 2021077029
(71) Applicant: Asahi Intecc Co., Ltd., Seto-shi, Aichi 489-0071 (JP)
(72) Inventor: KUBO, Yuta, Seto-shi, Aichi 489-0071 (JP); KAWAMURA, Kenta, Seto-shi, Aichi 489-0071 (JP)
(74) Representative: Prinz & Partner mbB
(86) International application number: PCT/JP2022/018173
(87) International publication number: WO 2022/230721

(57) **Abstract**

A catheter device includes a first catheter. The first catheter includes a first catheter shaft and an operating part that is attached to a proximal end portion of the first catheter shaft and operates the first catheter shaft. The operating part includes a rotary operation section that rotates the first catheter shaft about a central axis of the first catheter shaft, and a bending operation section that bends a distal end portion of the first catheter shaft.

## Description

### TECHNICAL FIELD

The technology disclosed in the present specification relates to a catheter device and a drug solution injection device.

### BACKGROUND ART

There is known a drug solution injection device that uses a catheter that can be inserted into a biological lumen to inject a drug solution into a patient's body. The drug solution injection device is required to improve operability of the catheter in order to accurately inject the drug solution in the desired position in the patient's body.

Conventionally, there is known a device capable of bending the distal end portion of the catheter by operating an operating part and thereby pulling an operation wire fixed to the distal end portion of the catheter (e.g., see Patent Literature 1). Further, there is known a drug solution injection device including a plunger that moves back and forth in a barrel storing the drug solution (e.g., see Patent Literature 2).

### CITATION LIST

### Patent Literature

Patent Literature 1: Japanese Patent No. 6221300
Patent Literature 2: Japanese Unexamined Patent Application Publication No. 2012-525869

### SUMMARY OF INVENTION

### TECHNICAL PROBLEM

There is room for improvement in operability of the conventional drug solution injection devices. It should be noted that such problems are not limited to drug solution injection devices but are common to catheter devices in general.

The present specification discloses a technology capable of solving the above-mentioned problems.

### SOLUTION TO PROBLEM

The technology disclosed in the present specification can be realized, for example, in the following aspects.
(1) A catheter device disclosed in the present specification includes a first catheter. The first catheter includes a first catheter shaft and an operating part that is attached to the proximal end portion of the first catheter shaft and operates the first catheter shaft. The operating part includes a rotary operation section that rotates the first catheter shaft about the central axis of the first catheter shaft, and a bending operation section that bends the distal end portion of the first catheter shaft.
   As described above, in the present catheter device, the operating part includes the rotary operation section that rotates the first catheter shaft and the bending operation section that bends the distal end portion of the first catheter shaft. Thus, the position and direction of the distal end portion of the first catheter shaft can be freely changed. Thus, according to the present catheter device, operability of the catheter device can be improved.
(2) In the above catheter device, the rotary operation section may be configured to include: a first operation input section that rotates the first catheter shaft about the central axis by rotating about the central axis in accordance with an operation by an operator and that is displaceable relative to the first catheter shaft in the central axis direction; and a rotary shaft that is attached to the first operation input section so as to rotate as the first operation input section rotates and that is attached to the proximal end portion of the first catheter shaft. According to the present catheter device, rotation of the first catheter shaft can be achieved by an intuitive and simple operation of the operator, making it possible to improve the operability of the catheter device.
(3) In the above catheter device, the bending operation section may be configured to include: a sliding member attached to the rotary shaft so as to be slidable in the central axis direction; an operation wire that connects the sliding member and the distal end portion of the first catheter shaft; a second operation input section that rotates in accordance with the operator's operation, and a transmission mechanism that converts a rotational force of the second operation input section into a force for sliding the sliding member, thereby transmitting the force to the sliding member. According to the present catheter device, it is possible to bend the distal end portion of the first catheter shaft by a simple operation of the operator, thereby improving the operability of the catheter device.
(4) In the above catheter device, the sliding member may be configured such that the rotary shaft maintains a state of receiving the force for sliding the sliding member from the transmission mechanism while rotating about the central axis. According to the present catheter device, it is possible to perform the bending operation of the distal end portion of the first catheter shaft regardless of the rotation angle of the first catheter shaft, thereby effectively improving the operability of the catheter device.
(5) In the above catheter device, the sliding member may be configured to include: a core member attached to the rotary shaft so as to rotate as the rotary shaft rotates about the central axis; and a rack member attached to the core member so as to be rotatable relative to the core member around the central axis and to slide as the core member slides in the central axis direction, the rack member having a plurality of teeth that mesh with a plurality of teeth of the transmission mechanism. According to the present catheter device, it is possible to reduce the number of places where the teeth are formed in the rack member, thereby achieving simplification and efficiency in processing.
(6) In the above catheter device, the sliding member may be configured to be a substantially cylindrical rack member having a plurality of teeth that mesh with the plurality of teeth of the transmission mechanism formed on the outer peripheral surface, the rack member being attached to the rotary shaft so as to rotate as the rotary shaft rotates around the central axis. According to the present catheter device, it is possible to reduce the number of parts in the operating part, thereby achieving simplification and efficiency in production.
(7) A drug solution injection device disclosed in the present specification includes: the catheter device as described above; and a second catheter including a second catheter shaft that is slidably inserted into the first catheter shaft via the operating part and has a drug solution storage lumen for storing a drug solution. In the present drug solution injection device, the first catheter shaft housing the second catheter shaft can be rotated by the rotary operation section of the operating part, and the distal end portion of the first catheter shaft housing the second catheter shaft can be bent by the bending operation section of the operating part, making it possible to freely change the position and direction of the distal end portion of the second catheter shaft having the drug solution storage lumen for storing the drug solution. Thus, according to the present drug solution injection device, it is possible to improve not only the operability of the drug solution injection device but also the accuracy of the drug solution injection position.
(8) The above drug solution injection device may be configured such that the rotary operation section includes: a first operation input section that rotates the first catheter shaft about the central axis by rotating about the central axis in accordance with the operator's operation and that is displaceable relative to the first catheter shaft in the central axis direction; and a rotary shaft that is attached to the first operation input section so as to rotate as the first operation input section rotates and that is attached to the proximal end portion of the first catheter shaft, in which the first operation input section is fixed to the second catheter directly or via another member, and the distal end of the second catheter is housed in the first catheter shaft when the first operation input section is at a first position, and the distal end of the second catheter protrudes from the distal end of the first catheter shaft when the first operation input section is at a second position on the distal end side from the first position. According to the present drug solution injection device, the first catheter shaft and the second catheter shaft can be moved to the drug solution injection position while the distal end of the second catheter shaft is housed in the first catheter shaft, making it possible to prevent the distal end of the second catheter shaft from damaging the inside of the patient's body. Further, according to the present drug solution injection device, the distal end of the second catheter shaft can protrude from the distal end of the first catheter shaft at the drug solution injection position by an intuitive and simple operation, thereby improving the operability of the drug solution injection device.
(9) The above drug solution injection device may be configured such that a needle portion is provided at the distal end of the second catheter shaft, the first operation input section of the rotary operation section is attached to the rotary shaft so as to rotate as the rotary shaft rotates about the central axis and to be displaceable relative to the rotary shaft in the central axis direction, and the needle portion of the second catheter is housed in the first catheter shaft when the first operation input section is at the first position, and the needle portion of the second catheter protrudes from the distal end of the first catheter shaft when the first operation input section is at the second position. According to the present drug solution injection device, the first catheter shaft and the second catheter shaft can be moved to a puncture position while the needle portion of the second catheter shaft is housed in the first catheter shaft, making it possible to prevent the needle portion from damaging the inside of the patient's body. Further, according to the present drug solution injection device, the needle portion can protrude from the distal end of the first catheter shaft at the puncture position by an intuitive and simple operation, thereby improving the operability of the drug solution injection device.
(10) The above drug solution injection device may be configured to further include: a wire slidably inserted into the drug solution storage lumen of the second catheter shaft and a plunger separate from the first catheter, in which the plunger includes a wire housing portion for housing the wire and a wire operation section that advances and retracts the wire in the drug solution storage lumen by sending out the wire from the wire housing portion or pulls the wire back into the wire housing portion. According to the present drug solution injection device, the operation of the first catheter shaft by the operating part of the second catheter and the drug solution ejection operation by the plunger can be performed by different operators, and as a result, the accuracy of both operations can be improved.
(11) The above drug solution injection device may be configured such that the wire operation section includes: a wire rack member that has a plurality of teeth and performs a rectilinear motion for a predetermined length; a roller pair that holds the wire therebetween and advances and retracts the wire by rotating; and a wire transmission mechanism that includes a plurality of gears including a driven gear having a plurality of teeth meshing with the plurality of teeth of the wire rack member and converts the rectilinear motion of the wire rack member into a rotary motion of the roller pair, thereby transmitting the motion to the roller pair. According to the present drug solution injection device, the wire can be advanced by a predetermined amount in the drug solution storage lumen by performing an operation that causes the wire rack member to perform the rectilinear motion for a predetermined length. As a result, a predetermined amount of the drug solution can be ejected from the distal end of the second catheter shaft, making it possible to control the ejection amount of the drug solution with high accuracy.
(12) The above drug solution injection device may be configured such that the plunger further includes a first biasing member that biases the wire rack member to be away from the driven gear. According to the present drug solution injection device, even if the wire rack member is unintentionally caused to perform the rectilinear motion, the driven gear does not rotate, making it possible to prevent the drug solution from being ejected due to an erroneous operation.
(13) The above drug solution injection device may be configured such that the plunger further includes a movement restricting portion that allows the wire rack member to move in a direction toward the driven gear and in a direction away from the driven gear when the wire rack member is at a start point and an end point of the rectilinear motion, and restricts the wire rack member from moving in the direction toward the driven gear or in the direction away from the driven gear when the wire rack member is at any other position. According to the present drug solution injection device, the wire rack member is prevented from being unintentionally separated from the driven gear while the wire rack member is engaged with the driven gear and performs the rectilinear motion. This can prevent that the ejection amount of the drug solution associated with one rectilinear motion of the wire rack member becomes less than the predetermined amount. Further, when the wire rack member that is not engaged with the driven gear is at a position other than the start point and the end point of the rectilinear motion, it is possible to prevent unintended engagement of the wire rack member with the driven gear and unintended ejection of the drug solution, making it possible to effectively improve the accuracy of the ejection amount of the drug solution.
(14) The above drug solution injection device may be configured such that the plunger further includes a second biasing member that biases the wire rack member toward the start point of the rectilinear motion. According to the present drug solution injection device, the operator does not need to perform an operation of moving the wire rack member to the position of the start point of the rectilinear motion. Thus, the operability of the drug solution injection device can be effectively improved.
(15) The above drug solution injection device may be configured such that the plunger further includes a counter portion that rotates and displays the number of times the wire rack member has performed the rectilinear motion; and a counter transmission mechanism that includes a plurality of gears including a driven gear having a plurality of teeth meshing with the plurality of teeth of the wire rack member and converts the rectilinear motion of the wire rack member into a rotary motion of the counter portion, thereby transmitting the motion to the counter portion. According to the present drug solution injection device, as the wire rack member performs the rectilinear motion, the counter portion rotates and updates the display of the number of times the rectilinear motion has been performed, making it possible to accurately recognize the number of times the wire rack member has performed the rectilinear motion, that is, the amount of the drug solution that has already been administered.
(16) The above drug solution injection device may be configured such that the wire transmission mechanism and the counter transmission mechanism share the driven gear. According to the present drug solution injection device, while achieving simplification of the configuration of the plunger, it is possible to achieve the ejection of the drug solution and the update of the display of the number of times each time the wire rack member performs the rectilinear motion.

Note that the technology disclosed in the present specification can be implemented in various forms. For example, the technology can be implemented in the form of a drug solution injection device, a catheter for drug solution injection, a plunger for drug solution injection, a system including the drug solution injection device, methods for producing these devices or systems, and the like.

### BRIEF DESCRIPTION OF DRAWINGS

FIG. 1 is an explanatory diagram schematically illustrating a configuration of a drug solution injection device 10 according to a first embodiment.
FIG. 2 is an explanatory diagram illustrating a configuration of a needle catheter 40.
FIG. 3 is an explanatory diagram illustrating a detailed configuration of an operation catheter 20.
FIG. 4 is an explanatory diagram illustrating the detailed configuration of the operation catheter 20.
FIG. 5 is an explanatory diagram illustrating the detailed configuration of the operation catheter 20.
FIG. 6 is an explanatory diagram illustrating an operation of the operation catheter 20.
FIG. 7 is an explanatory diagram illustrating the operation of the operation catheter 20.
FIG. 8 is an explanatory diagram illustrating the operation of the operation catheter 20.
FIG. 9 is an explanatory diagram illustrating the operation of the operation catheter 20.
FIG. 10 is an explanatory diagram illustrating a detailed configuration of a plunger 50.
FIG. 11 is an explanatory diagram illustrating the detailed configuration of the plunger 50.
FIG. 12 is an explanatory diagram illustrating the detailed configuration of the plunger 50.
FIG. 13 is an explanatory diagram illustrating the detailed configuration of the plunger 50.
FIG. 14 is an explanatory diagram illustrating the detailed configuration of the plunger 50.
FIG. 15 is an explanatory diagram illustrating an operation of the plunger 50.
FIG. 16 is an explanatory diagram schematically illustrating a configuration of an operation catheter 20a according to a second embodiment.

### EMBODIMENTS OF THE INVENTION

### A. First embodiment:

A-1. Configuration of drug solution injection device 10:

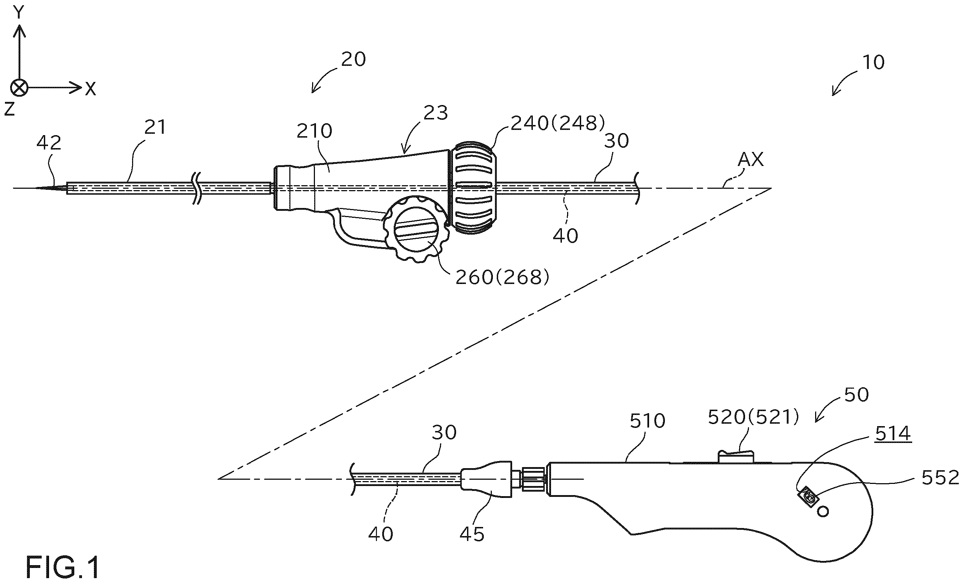

FIG. 1 is an explanatory diagram schematically showing a configuration of a drug solution injection device 10 according to a first embodiment. FIG. 1 shows a side configuration of the drug solution injection device 10. Note that, in FIG. 1, illustration of a part of the drug solution injection device 10 is omitted. In FIG. 1, mutually orthogonal XYZ axes are shown. The X axis corresponds to a length direction of the drug solution injection device 10, the Y axis corresponds to a height direction of the drug solution injection device 10, and the Z axis corresponds to a width direction of the drug solution injection device 10. In FIG. 1, a negative direction side of the X axis is the distal end side (far side) to be inserted into the body, and a positive direction side of the X-axis is the proximal end side (near side) operated by an operator such as a physician. The same applies to the other drawings. Hereinafter, with regard to the drug solution injection device 10 and its constituent members, the end on the distal end side is referred to as "distal end", and the distal end and the vicinity thereof are referred to as "distal end portion", while the end on the proximal end side is referred to as "proximal end", and the proximal end and the vicinity thereof are referred to as "proximal end portion". Further, in the following, for convenience of explanation, a positive direction side of the Y-axis is referred to as "upper side" and a negative direction side of the Y-axis is referred to as "lower side".

The drug solution injection device 10 is a medical device used for injecting a drug solution into the body of a patient using a catheter that can be inserted into a biological lumen. The term "biological lumen" described herein includes various lumens such as a blood vessel system, a lymphatic system, a biliary system, a urinary system, a respiratory system, a digestive system, a secretory gland, and a reproductive organ.

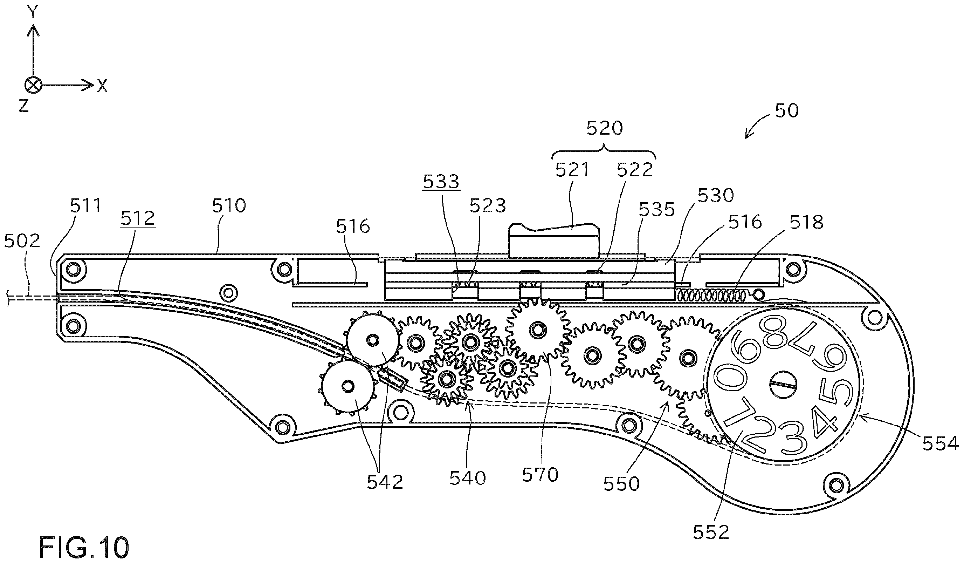

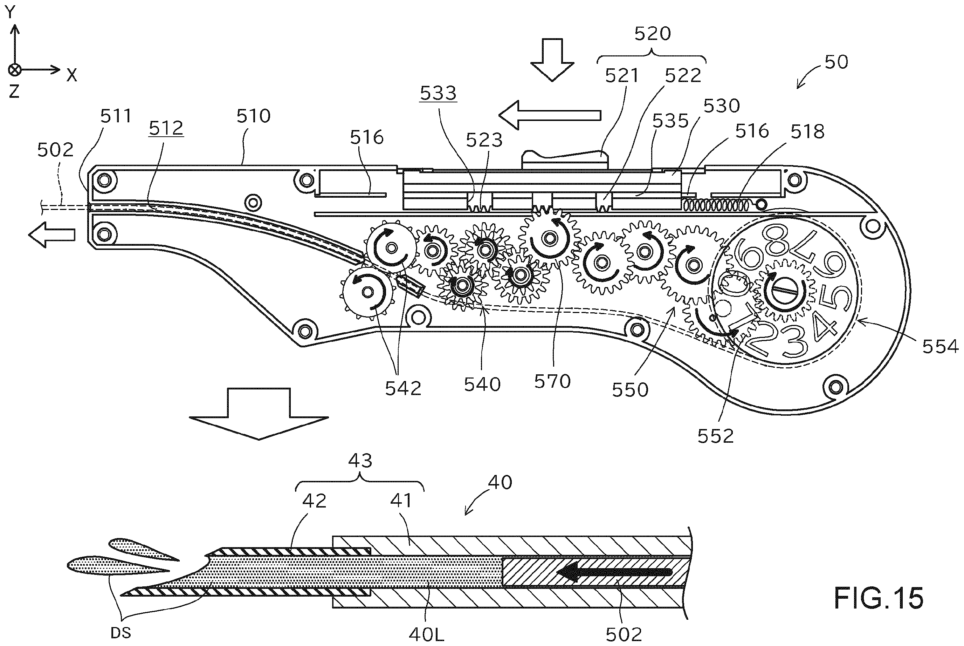

The drug solution injection device 10 includes an operation catheter 20, a relay catheter 30, a needle catheter 40, a plunger 50, and a wire 502 (see FIGS. 10, 15, etc.). In the drug solution injection device 10, the distal end portion of the relay catheter 30 is fixed to a first operation input section 240 provided at the proximal end portion of an operating part 23 constituting the operation catheter 20. The proximal end portion of the relay catheter 30 is fixed to a connector 45 of the needle catheter 40. That is, the needle catheter 40 is connected to the operation catheter 20 via the relay catheter 30. The operation catheter 20 is an example of a first catheter in the claims, and the needle catheter 40 is an example of a second catheter in the claims.

Note that FIG. 1 shows a central axis AX of the operation catheter 20 (more specifically, a sheath 21 of the operation catheter 20, which is described below). In the posture of the drug solution injection device 10 shown in FIG. 1, the central axis AX is parallel to the X axis. Further, the central axes of the relay catheter 30 and the needle catheter 40 substantially coincide with the central axis AX of the operation catheter 20.

A-2. Configuration of needle catheter 40:

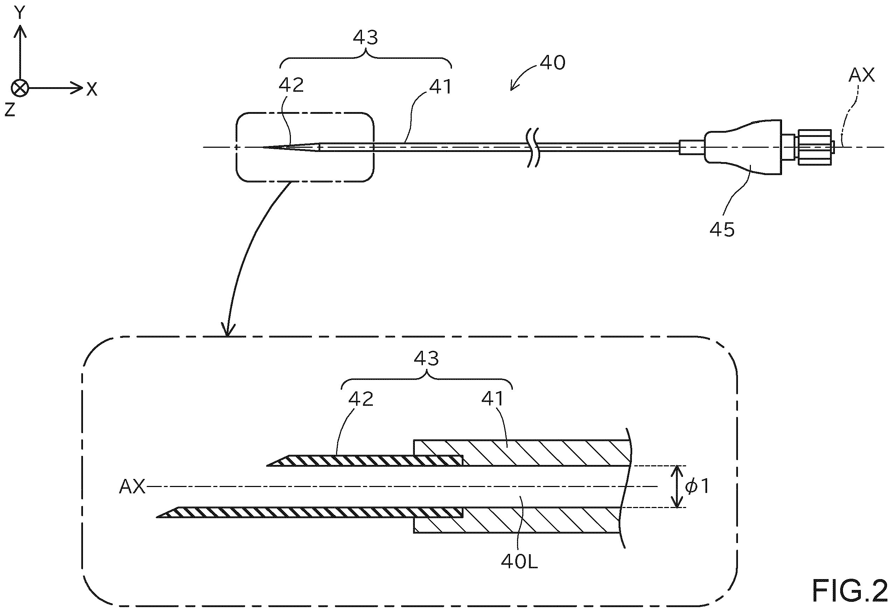

FIG. 2 is an explanatory diagram showing a configuration of the needle catheter 40. The upper part of FIG. 2 shows the side configuration of the needle catheter 40, and the lower part of FIG. 2 shows the cross-sectional configuration of the distal end portion of the needle catheter 40.

The needle catheter 40 is a device used to be inserted into the biological lumen of the patient, carry a drug solution to a desired position in the patient's body, and inject the drug solution in the desired position. The needle catheter 40 includes a catheter shaft 43 and a connector 45. The catheter shaft 43 is an example of a second catheter shaft in the claims.

The catheter shaft 43 is an elongated member extending along the central axis AX. More specifically, the catheter shaft 43 is a substantially cylindrical member having openings formed at the distal end and the proximal end, and having a lumen communicating with both openings formed inside. The lumen of the catheter shaft 43 functions as a drug solution storage lumen 40L for storing a drug solution. The cross section of the drug solution storage lumen 40L is formed, for example, in a substantially circular shape with a substantially constant inner diameter ϕ1.

The catheter shaft 43 includes a shaft main body portion 41 and a needle portion 42. The proximal end portion of the needle portion 42 is joined to the distal end portion of the shaft main body portion 41 by, for example, adhesion. A sharp needle tip is formed at the distal end portion of the needle portion 42.

The shaft main body portion 41 preferably has antithrombogenicity, flexibility, and biocompatibility, and can be formed of a resin material or a metal material. As the resin material for forming the shaft main body portion 41, for example, a polyamide resin, a polyolefin resin, a polyester resin, a polyurethane resin, a silicon resin, a fluororesin, or the like can be adopted. Further, as the metal material for forming the shaft main body portion 41, for example, stainless steel such as SUS304, a NiTi alloy, a cobalt chromium alloy, or the like can be adopted. Note that, in order to improve at least some of flexibility, torque conveyance property, push-in property, kink resistance, blood vessel followability, lesion penetration, and operability, a coiled body or a braided body may be embedded in the thick portion of the shaft main body portion 41. Further, the shaft main body portion 41 may be configured by a plurality of layers formed of the same or different materials. Further, the needle portion 42 preferably has antithrombogenicity and biocompatibility, and can be formed of a metal material such as, for example, stainless steel such as SUS304, a NiTi alloy, or a cobalt chromium alloy.

The connector 45, which is connected to the proximal end portion of the catheter shaft 43, connects the needle catheter 40 and the plunger 50 (FIG. 1) and functions as a valve that seals the drug solution storage lumen 40L of the needle catheter 40. The connector 45 can be formed of a resin material such as, for example, polyurethane, polypropylene, or rigid polyvinyl chloride.

Note that the proximal end portion of the relay catheter 30 (FIG. 1) is fixed to the connector 45. The relay catheter 30 is a member for protecting the needle catheter 40 and is a substantially cylindrical member having openings formed at the distal end and the proximal end, and having a lumen communicating with both openings formed inside. The needle catheter 40 is housed in the lumen of the relay catheter 30. The relay catheter 30 can be formed of a resin material such as, for example, PTFE.

A-3. Configuration of operation catheter 20:

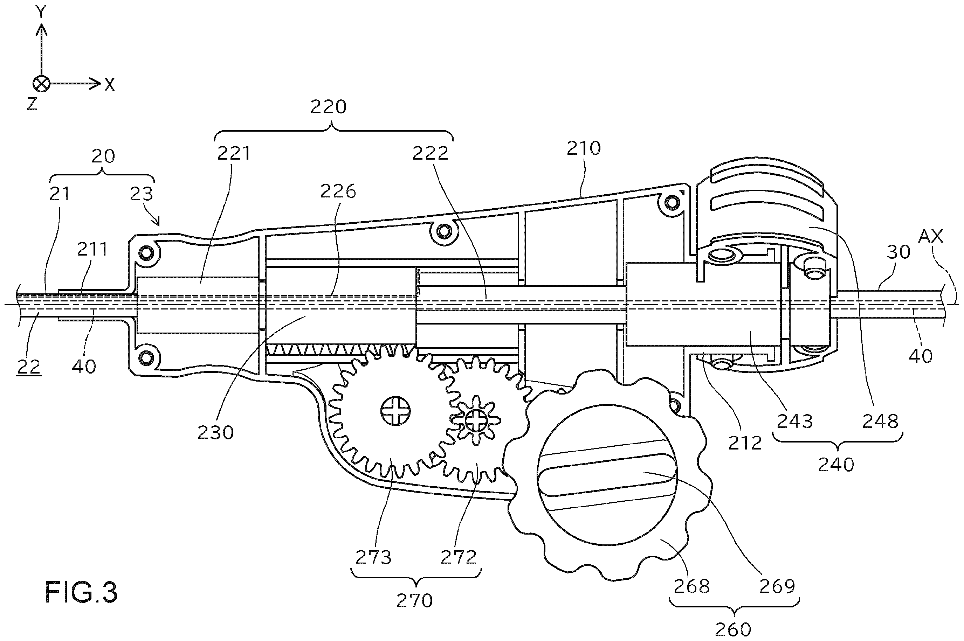

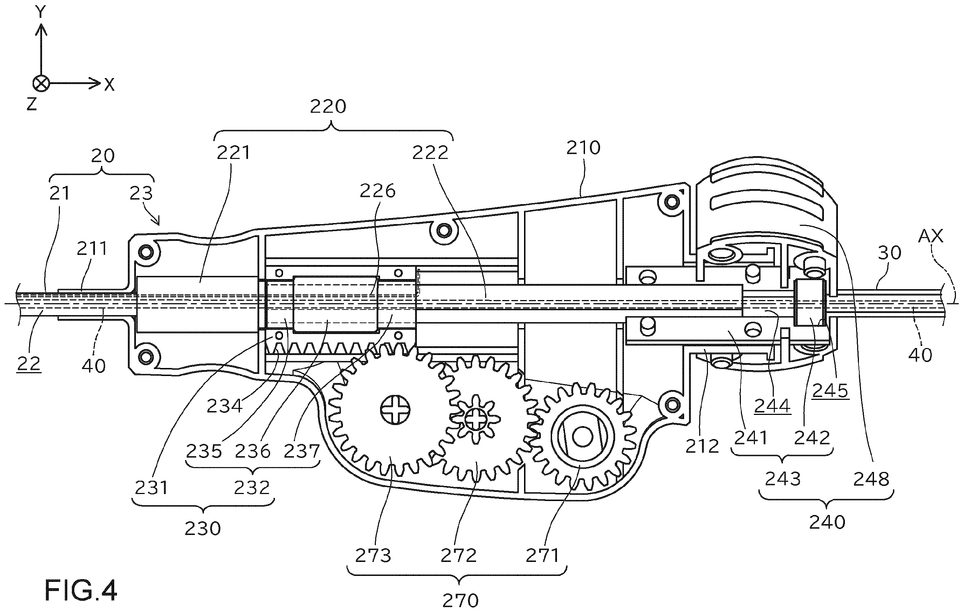

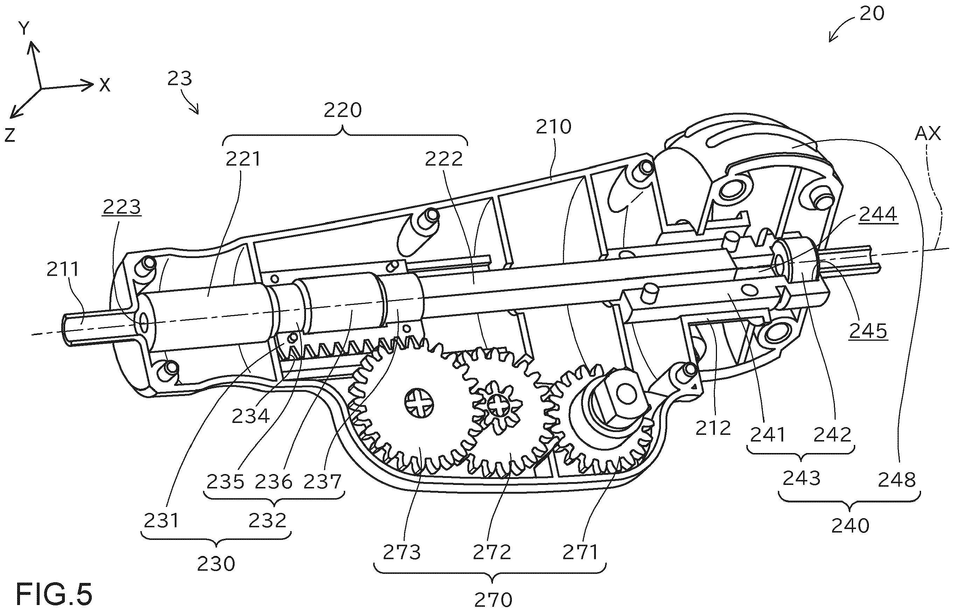

FIG. 3 to FIG. 5 are explanatory diagrams showing a detailed configuration of the operation catheter 20. FIG. 3 to FIG. 5 show an internal configuration of the operation catheter 20 while omitting a part of the operation catheter 20. FIG. 3 shows the configuration of the operation catheter 20 in a side view (viewed from the Z-axis direction). In FIG. 3, a part of a housing 210 (a front side part in the drawing) and a part of a first dial 248 (a lower front side part in the drawing) of the operation catheter 20 are omitted from the drawing. FIG. 4 shows the configuration of the operation catheter 20 in the side view (viewed from the Z-axis direction). In FIG. 4, in addition to the parts omitted in FIG. 3, a part of a sliding member 230 (a front side part in the drawing), a part of a connecting member 243 (a front side part in the drawing), and a second operation input section 260 of the operation catheter 20 are omitted from the drawing. FIG. 5 shows a perspective configuration of the operation catheter 20 while omitting, from the drawing, the same parts of the operation catheter 20 as those omitted in FIG. 4.

The operation catheter 20 is a device that performs rotary and bending operations of the needle catheter 40. The operation catheter 20 includes a sheath 21 and an operating part 23. Note that, in FIG. 5, illustration of the sheath 21 is omitted. The sheath 21 is an example of a first catheter shaft in the claims.

The sheath 21 is an elongated member extending along the central axis AX. More specifically, the sheath 21 is a substantially cylindrical member having openings formed at the distal end and the proximal end, and having a lumen 22 communicating with both openings formed inside. The cross section of the lumen 22 of the sheath 21 is formed in, for example, a substantially circular shape with an inner diameter larger than the outer diameter of catheter shaft 43 of the needle catheter 40. The catheter shaft 43 of the needle catheter 40 is slidably inserted into the lumen 22 of the sheath 21 via the operating part 23. Further, the distal end portion of the sheath 21 is more flexible than other parts.

The sheath 21 preferably has antithrombogenicity, flexibility, and biocompatibility, and can be formed of a resin material or a metal material. As a material for forming the sheath 21, the same material as the material for forming the shaft main body portion 41 of the needle catheter 40 described above can be adopted. Further, a coiled body or a braided body may be embedded in the thick portion of the sheath 21 in the same manner as the shaft main body portion 41. Further, as with the shaft main body portion 41, the sheath 21 may be configured by multiple layers formed of the same or different materials.

The operating part 23 is a device for operating the sheath 21 and is attached to the proximal end portion of the sheath 21. As shown in FIG. 3 to FIG. 5, the operating part 23 includes a housing 210, a rotary shaft 220, a sliding member 230, a first operation input section 240, a second operation input section 260, and a transmission mechanism 270. Each part of the operating part 23 can be formed of, for example, a resin material or a metal material.

The housing 210 is a case that houses each member that constitutes the operating part 23 inside. Note that, in FIG. 3 to FIG. 5, illustration of a part of the housing 210 is omitted. The housing 210 has a substantially conical shape with a smaller outer diameter toward the distal end side. At the distal end of the housing 210, a tubular distal end side guide portion 211 having an inner diameter slightly larger than the outer diameter of the sheath 21 is formed. Further, at the proximal end of the housing 210, a cylindrical proximal end side guide portion 212 having an inner diameter slightly larger than the outer diameter of a connection main body portion 241, which is described later, is formed.

The rotary shaft 220 is an elongated member extending along a central axis AX. More specifically, the rotary shaft 220 is a tubular member having openings formed at the distal end and the proximal end, and having a lumen 223 (see FIG. 5) communicating with both openings formed inside. The cross-section of the lumen 223 of the rotary shaft 220 is formed in, for example, a substantially circular shape with an inner diameter larger than the outer diameter of the catheter shaft 43 of the needle catheter 40. The lumen 223 of the rotary shaft 220 communicates with the lumen 22 of the sheath 21, and the needle catheter 40 is slidably inserted into these lumens 223 and 22.

The rotary shaft 220 is supported by the housing 210 so as to be rotatable about the central axis AX. The proximal end portion of the sheath 21 and a distal end portion 221 of the rotary shaft 220 are fixed while the proximal end portion of the sheath 21 is inserted into the lumen 223 in the distal end portion 221 of the rotary shaft 220. Thus, when the rotary shaft 220 rotates about the central axis AX, the sheath 21 also rotates about the central axis AX synchronously. Further, the relative position of the rotary shaft 220 with respect to the housing 210 is fixed in the central axis AX direction. Note that an outer shape of the cross section of the distal end portion 221 of the rotary shaft 220 is a substantially circular shape, and an outer shape of the cross section of the proximal end portion 222 of the rotary shaft 220 is a substantially rectangular shape.

The sliding member 230 is a substantially cylindrical member and has a substantially rectangular cross-sectional outer shape. The sliding member 230 is attached to the rotary shaft 220 so as to be slidable in the central axis AX direction. More specifically, the sliding member 230 is configured by a core member 232 and a rack member 231 (see FIG. 4 and FIG. 5. Note that, in FIG. 4 and FIG. 5, illustration of a part of the rack member 231 is omitted). The core member 232 has a lumen having substantially the same shape as the outer shape of the proximal end portion 222 of the rotary shaft 220. The core member 232 is fixed to the rotary shaft 220 with the proximal end portion 222 of the rotary shaft 220 housed in the lumen of the core member 232. Thus, when the rotary shaft 220 rotates about the central axis AX, the core member 232 also rotates about the central axis AX in synchronization. Further, the core member 232 is configured by a distal end portion 235, a proximal end portion 237, and an intermediate portion 236 sandwiched between the distal end portion 235 and the proximal end portion 237. The outer shape of the cross-section of each of these portions is a substantially circular shape. However, the outer diameter of the intermediate portion 236 is larger than the outer diameters of the distal end portion 235 and the proximal end portion 237.

The rack member 231 has a lumen having substantially the same shape as the outer shape of the core member 232. The core member 232 is fixed to the rack member 231 while being housed in the lumen of the rack member 231. The rack member 231 is rotatable relative to the core member 232 about the central axis AX. On the other hand, in the central axis AX direction, the rack member 231 slides as the core member 232 slides in the central axis AX direction by an engagement between the outer circumferential surface of the core member 232 and the inner circumferential surface of the rack member 231. That is, the rack member 231 is attached to the core member 232 so as to be rotatable relative to the core member 232 about the central axis AX and to slide as the core member 232 slides in the central axis AX direction. A plurality of teeth 234 are formed on a part of the outer peripheral surface of the rack member 231 (specifically, the lower surface, i.e., the surface facing a transmission mechanism 270 described below).

Further, the proximal end portion of an operation wire 226 is fixed to the proximal end portion of the sliding member 230. The operation wire 226 extends from a part of the sliding member 230 to which the operation wire 226 is fixed, through the lumen 223 of the rotary shaft 220 and the lumen 22 of the sheath 21, to the flexible distal end portion of the sheath 21, and is fixed to the sheath 21 at this position. That is, the operation wire 226 connects the sliding member 230 and the distal end portion of the sheath 21. Note that the number of operation wires 226 to be installed may be one or more.

The first operation input section 240 is a portion that receives an operation by an operator (user) such as a physician, and is provided in the proximal end portion of the operating part 23. The first operation input section 240 includes a connecting member 243 and a first dial 248.

The connecting member 243 includes the connection main body portion 241 and an annular member 242. The connection main body portion 241 is a substantially cylindrical member having a lumen 244. The distal end portion of the connection main body portion 241 is housed in the housing 210, and the remaining part of the connection main body portion 241 protrudes outside the housing 210. The shape of the lumen 244 in the distal end portion of the connection main body portion 241 is substantially the same as the outer shape of the proximal end portion 222 of the rotary shaft 220, and the proximal end portion 222 of the rotary shaft 220 is fitted into the lumen 244. Thus, when the connection main body portion 241 rotates about the central axis AX, the rotary shaft 220 also rotates about the central axis AX synchronously. Further, the connection main body portion 241 is slidable in the central axis AX direction with respect to the rotary shaft 220.

The lumen 244 of the connection main body portion 241 includes an enlarged diameter portion 245 with a large outer shape, and the annular member 242 is housed in this enlarged diameter portion 245. The outer diameter of the annular member 242 is larger than the inner diameter of the enlarged diameter portion 245 and larger than the inner diameters of parts of the lumen 244 other than the enlarged diameter portion 245. Thus, the annular member 242 is rotatable relative to the connection main body portion 241 about the central axis AX, and slides as the connection main body portion 241 slides in the central axis AX direction. Further, the distal end portion of the relay catheter 30 is fixed to the annular member 242.

The first dial 248 is a member that rotates about the central axis AX by a rotary operation by the operator. The first dial 248 is non-rotatably and non-slidably attached to a part of the connection main body portion 241 that protrudes outside the housing 210. Thus, when the first dial 248 rotates about the central axis AX, the connection main body portion 241 also rotates about the central axis AX in synchronously, and when the first dial 248 slides in the central axis AX direction, the connection main body portion 241 also slides in the central axis AX direction synchronously.

The second operation input section 260 is a portion that receives an operation by the operator, and is provided at the proximal end portion of the operating part 23. The second operation input section 260 includes a second dial 268 and a stopper 269.

The second dial 268 is a member that rotates about an axis (the Z-axis) in the direction perpendicular to the central axis AX by the rotary operation by the operator. Further, the stopper 269 slides against the second dial 268 to switch between a state that allows the second dial 268 to rotate and a state that prevents the second dial 268 from rotating.

The transmission mechanism 270 is a mechanism that converts the operator's force for rotating the second dial 268 of the second operation input section 260 into a force for sliding the sliding member 230, thereby transmitting the force to the sliding member 230. In other words, the transmission mechanism 270 is a mechanism that converts a rotary motion of the second dial 268 about the Z-axis into a rectilinear motion of the sliding member 230 in the central axis AX direction. The transmission mechanism 270 is configured by a plurality of (three) gears 271, 272, and 273 that mesh with each other. The first gear 271 of the transmission mechanism 270 rotates as the second dial 268 rotates. Further, a plurality of teeth of the third gear 273 mesh with the plurality of teeth 234 of the rack member 231 of the sliding member 230. Thus, when the third gear 273 rotates, the sliding member 230 slides in the central axis AX direction while being supported by the rotary shaft 220. Note that, in the present embodiment, a reduction ratio by the transmission mechanism 270 is set to, for example, about 1:2 to 1:4 so that the operation load of the second operation input section 260 can be reduced. That is, it is set to satisfy the following: the movement distance of the sliding member 230/the rotation distance of the teeth of the first gear 271=about 1/2 to 1/4.

A-4. Operation of operation catheter 20:

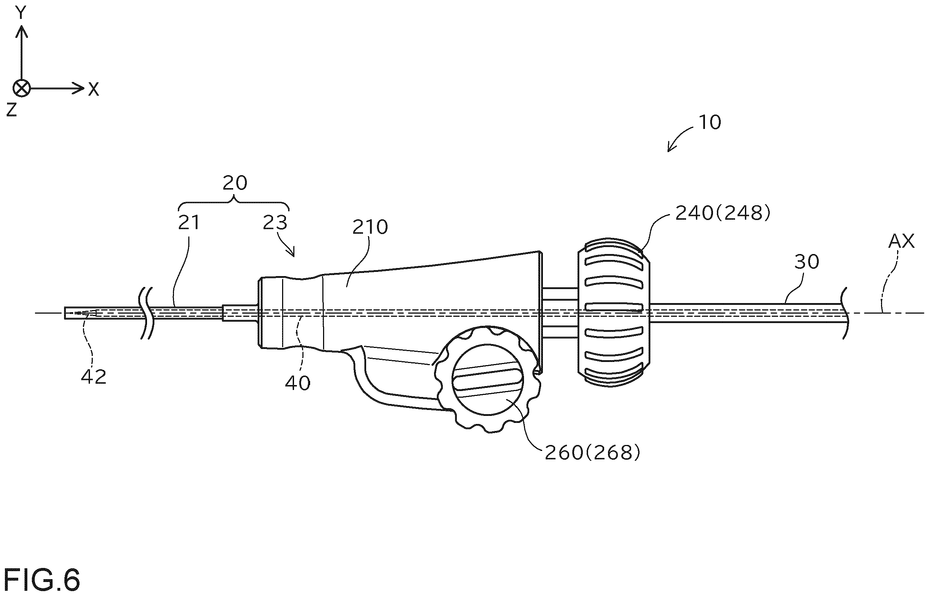

FIG. 6 to FIG. 9 are explanatory diagrams showing the operation of the operation catheter 20. In the initial state shown in FIG. 6, the first dial 248 constituting the first operation input section 240 of the operating part 23 is away from the housing 210 toward the proximal end side. In this state, the needle portion 42 of the needle catheter 40 housed in the lumens of the operation catheter 20 and the relay catheter 30 is positioned near the distal end of the sheath 21 without protruding from the sheath 21. The position of the first operation input section 240 of the operating part 23 in the initial state shown in FIG. 6 is an example of a first position in the claims.

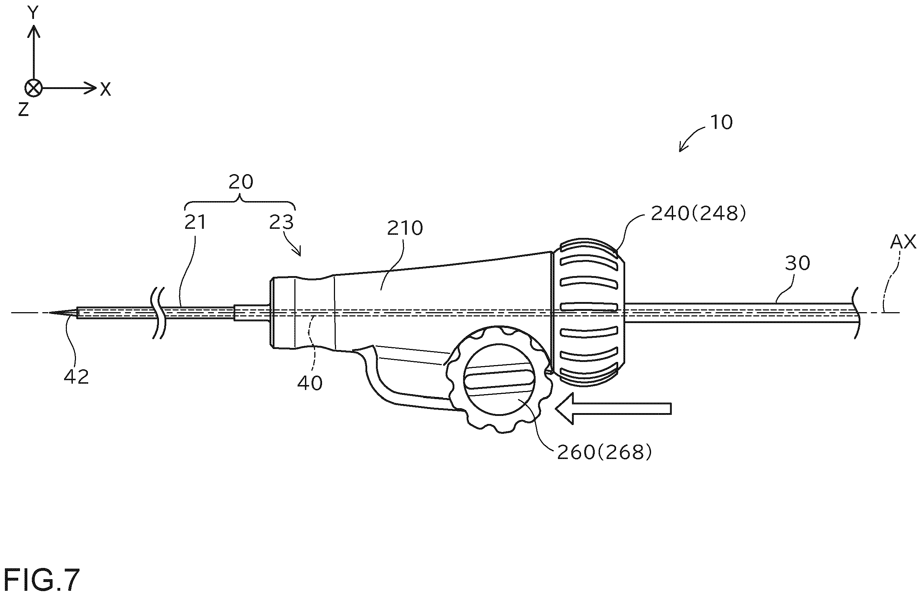

In the initial state, when the operator holds the first dial 248 constituting the first operation input section 240 and performs an operation of pushing it toward the distal end side, as shown in FIG. 7, the needle portion 42 of the needle catheter 40 protrudes from the distal end of the sheath 21. That is, as described above, the sheath 21 is fixed to the rotary shaft 220 (see FIG. 3, etc.) of the operating part 23, and the position of the rotary shaft 220 with respect to the housing 210 is fixed in the central axis AX direction, thus the position of the sheath 21 with respect to the housing 210 is also fixed. On the other hand, since the first operation input section 240 is slidable in the central axis AX direction with respect to the rotary shaft 220, it is also slidable in the central axis AX direction with respect to the housing 210. Further, the distal end portion of the relay catheter 30 is fixed to the annular member 242 of the first operation input section 240, and the proximal end portion of the relay catheter 30 is fixed to the connector 45 of the needle catheter 40. Thus, when the first operation input section 240 moves toward the distal end relative to the housing 210, the needle catheter 40 also moves toward the distal end side relative to the housing 210 by the same amount. As a result, the needle catheter 40 moves toward the distal end side relative to the sheath 21, and the needle portion 42 of the needle catheter 40 protrudes from the distal end of the sheath 21. The position of the first operation input section 240 of the operating part 23 in the state shown in FIG. 7 is an example of a second position in the claims.

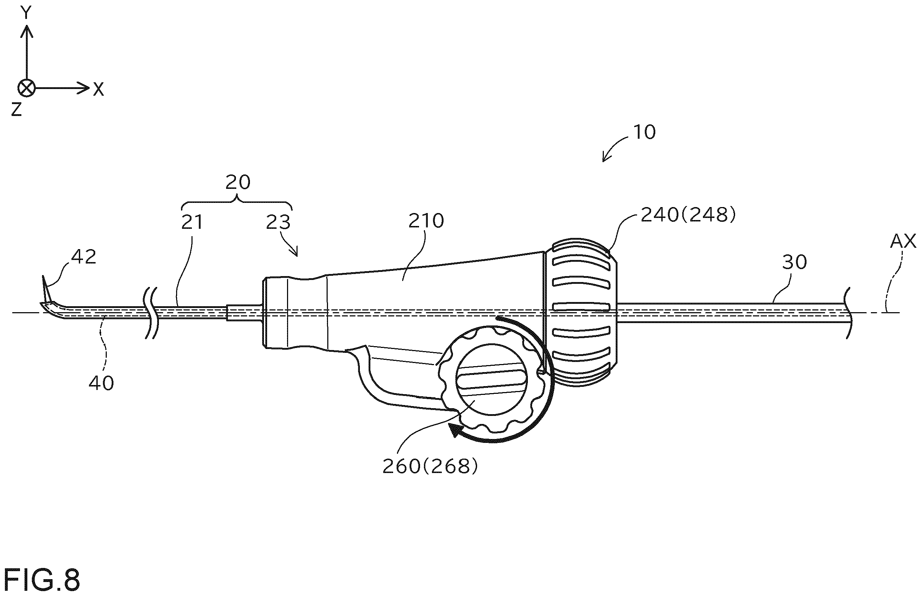

Further, when the operator holds the second dial 268 constituting the second operation input section 260 and performs the rotary operation, as shown in FIG. 8, the distal end portion of the sheath 21 bends, and accordingly the distal end portion of the needle catheter 40 housed in the sheath 21 also bends. That is, as described above, when the second dial 268 of the second operation input section 260 rotates, the rotational force applied to the second dial 268 is converted to the force for sliding the sliding member 230 by the transmission mechanism 270 (see FIG. 3, etc.), thereby transmitting the force to the sliding member 230. Thus, when the second dial 268 is rotated in a rotational direction so as to move the sliding member 230 toward the proximal end side, the sliding member 230 moves to the proximal end side, and the tension of the operation wire 226 stretched between the sliding member 230 and the distal end portion of the sheath 21 increases. As a result, the flexible distal end portion of the sheath 21 bends. Conversely, when the second dial 268 is rotated in a rotational direction so as to move the sliding member 230 toward the distal end side, the sliding member 230 moves toward the distal end side, and the tension of the operation wire 226 is reduced. As a result, the distal end portion of the sheath 21 returns to the straight shape. Note that, sliding the stopper 269 of the second operation input section 260 against the second dial 268 can cause a state that prevents the rotation of the second dial 268. In this manner, the bent state of the distal end portion of the sheath 21 can be maintained. Further, the bending operation of the sheath 21 can be performed in the same manner, whether the needle portion 42 does not protrude from the distal end of the sheath 21 or the needle portion 42 protrudes from the distal end of the sheath 21. The second operation input section 260, the transmission mechanism 270, the sliding member 230, and the operation wire 226 of the operating part 23 are examples of a bending operation section in the claims.

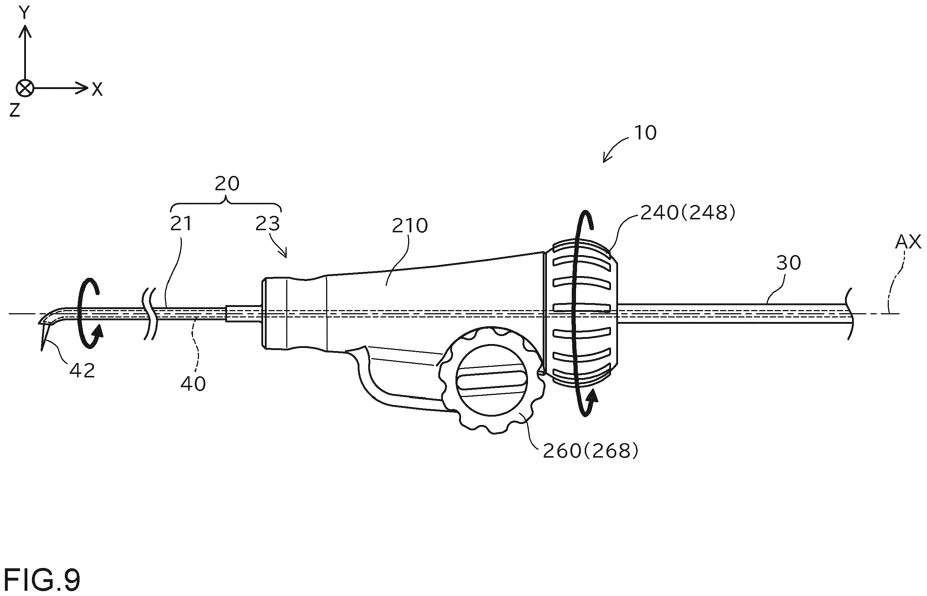

Further, when the operator holds the first dial 248 constituting the first operation input section 240 and performs the rotary operation, as shown in FIG. 9, the sheath 21 rotates about the central axis AX, and accordingly the needle catheter 40 housed in the sheath 21 also rotates about the central axis AX. That is, as described above, when the first dial 248 of the first operation input section 240 rotates about the central axis AX, the connection main body portion 241 (see FIG. 3, etc.) also rotates about the central axis AX synchronously. Accordingly, the rotary shaft 220 also rotates about the central axis AX synchronously, and the sheath 21 fixed to the rotary shaft 220 also rotates about the central axis synchronously. Note that the rotational direction of the sheath 21 is either clockwise or counterclockwise depending on the rotational direction of the first dial 248. Further, the rotary operation of the sheath 21 can be performed in the same manner, whether the needle portion 42 does not protrude from the distal end of the sheath 21 or the needle portion 42 protrudes from the distal end of the sheath 21. Further, as described above, when the rotary shaft 220 rotates about the central axis AX, the core member 232 of the sliding member 230 also rotates about the central axis AX synchronously. However, since the rack member 231 of the sliding member 230 can rotate relative to the core member 232 about the central axis AX, even if the rotary shaft 220 rotates, the rack member 231 maintains the state of receiving the force for sliding the sliding member 230 from the transmission mechanism 270. Thus, by simultaneously operating the first dial 248 and the second dial 268, the rotary operation of the sheath 21 and the bending operation of the sheath 21 can be performed in parallel. The first operation input section 240 and the rotary shaft 220 of the operating part 23 are an example of a rotary operation section in the claims.

A-5. Configuration of plunger 50:

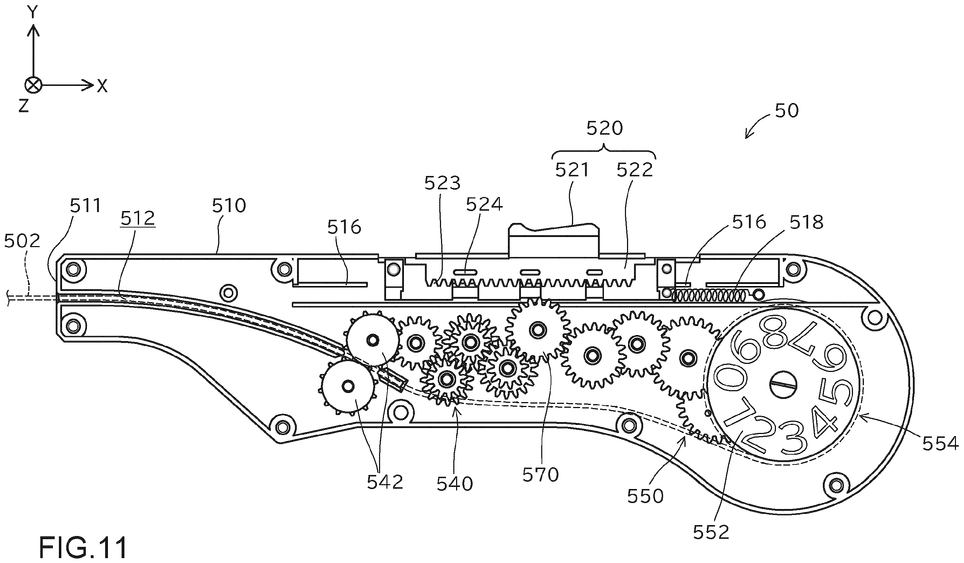

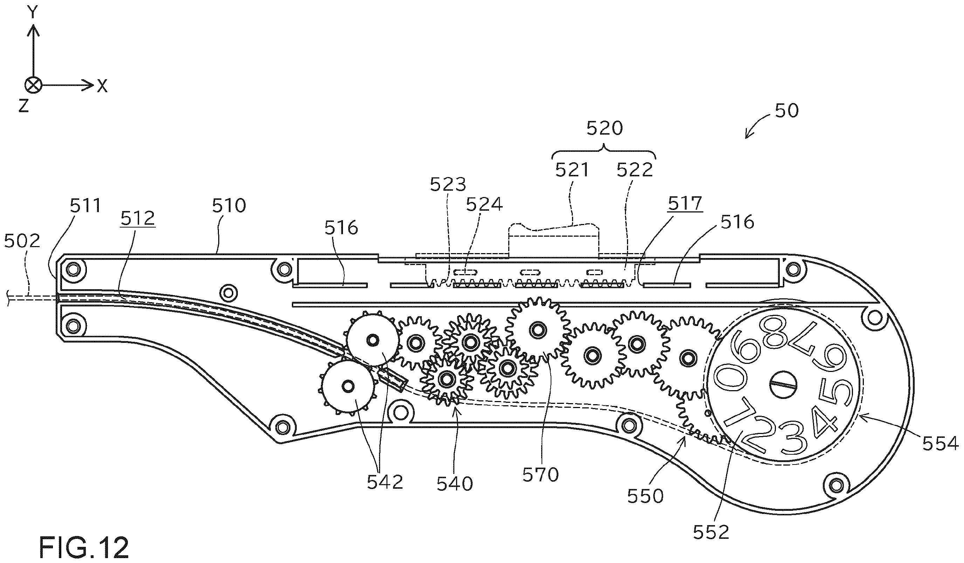

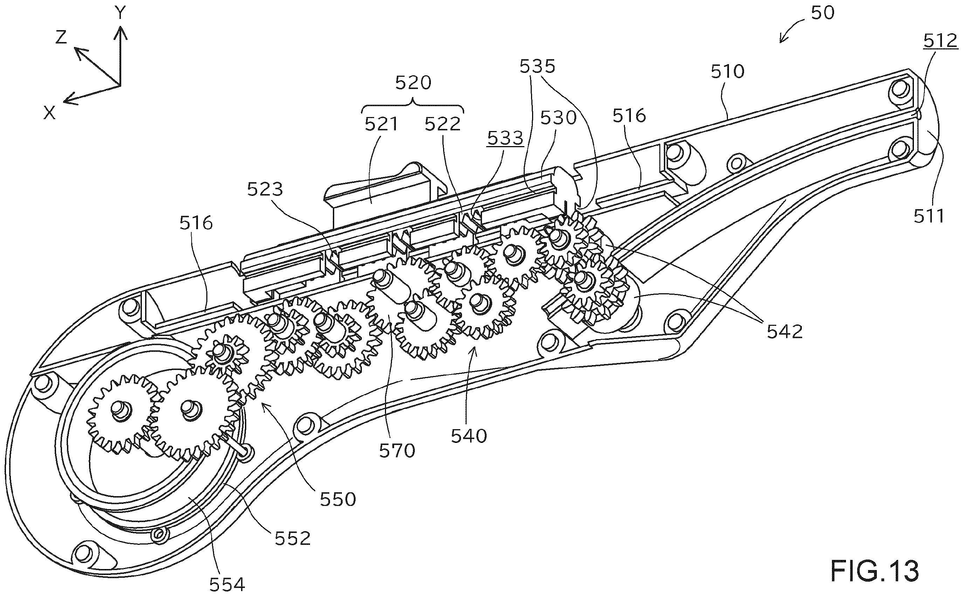

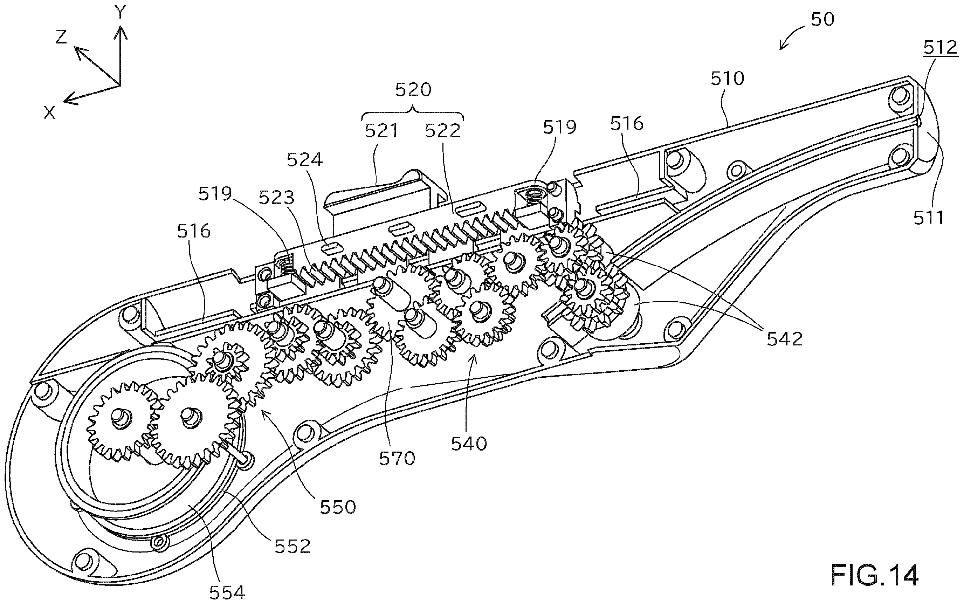

FIG. 10 to FIG. 14 are explanatory diagrams showing a detailed configuration of the plunger 50. FIG. 10 to FIG. 14 show an internal configuration of the plunger 50 while omitting a part of the plunger 50. FIG. 10 shows a configuration of the plunger 50 in a side view (viewed from the Z-axis direction). In FIG. 10, a part of the housing 510 (a front side part in the drawing) is omitted from illustration of the plunger 50. FIG. 11 shows the configuration of the plunger 50 in a side view (viewed in the Z-axis direction). In FIG. 11, in addition to the part omitted in FIG. 10, a part of a rack case 530 (a front side part in the drawing) is omitted from illustration of the plunger 50. FIG. 12 shows the configuration of the plunger 50 in a side view (viewed in the Z-axis direction). In FIG. 12, in addition to the parts omitted in FIG. 11, a wire rack member 520 is omitted from illustration of the plunger 50 (however, the position of the wire rack member 520 is indicated by a dashed line). FIG. 13 shows a perspective configuration of the plunger 50. In FIG. 13, the same part of the plunger 50 as that omitted in FIG. 10 is omitted from illustration. FIG. 14 shows the perspective configuration of the plunger 50. In FIG. 14, the same parts of the plunger 50 as those omitted in FIG. 11 are omitted from illustration.

The plunger 50 is a device that advances the wire 502 in the drug solution storage lumen 40L (see FIG. 2) of the needle catheter 40 to eject the drug solution stored in the drug solution storage lumen 40L from the distal end of the needle portion 42. The plunger 50 is a separate device from the operation catheter 20.

Note that the wire 502 is an elongated member having a substantially constant outer diameter from the distal end to the proximal end. The wire 502 has a substantially circular cross-sectional shape. The outer diameter of the wire 502 is substantially the same (clearance: 100 µm or less) as the inner diameter ϕ1 (see FIG. 2) of the drug solution storage lumen 40L formed in the catheter shaft 43 of the needle catheter 40. The wire 502 preferably has antithrombogenicity, flexibility, and biocompatibility, and can be formed of a metal material such as, for example, stainless steel such as SUS304, or a NiTi alloy. The wire 502 may be coated with a resin or the like. Note that, in FIG. 13 and FIG. 14, the wire 502 is omitted from illustration.

The plunger 50 includes the housing 510, a bobbin 554, the wire rack member 520, the rack case 530, a roller pair 542, a wire transmission mechanism 540, a counter portion 552, and a counter transmission mechanism 550. Each part of the plunger 50 can be formed of, for example, a resin material or a metal material.

The housing 510 is a case that houses each member constituting the plunger 50 in its inside. In FIG. 10 to FIG. 14, a part of the housing 510 is omitted from illustration. The shape of the housing 510 is, for example, slightly elongated in the X-axis direction, so that it can be easily held with the thumb placed on the upper surface of the wire rack member 520. A guide groove 512 communicating with a hole opening in a distal end surface 511 of the housing 510 is formed inside the distal end portion of the housing 510 to guide the wire 502.

The bobbin 554 is a substantially cylindrical member that is rotatably attached in the vicinity of the proximal end portion inside the housing 510 about a rotation axis in a predetermined direction (the Z-axis direction in the present embodiment). The wire 502 is wound around the outer peripheral surface of the bobbin 554 so that the bobbin 554 houses a part of the wire 502 on the proximal end side. Note that the wire 502 extends from the position of the bobbin 554 to the distal end side, passes through the roller pair 542 and the guide groove 512, protrudes from the hole formed in the distal end surface 511 of the housing 510, and is slidably inserted into the drug solution storage lumen 40L (see FIG. 2) of the needle catheter 40 via the connector 45 (see FIG. 1) of the needle catheter 40. The bobbin 554 is an example of a wire housing portion in the claims.

The wire rack member 520 includes a substantially rectangular parallelepiped proximal end portion 522 elongated in a predetermined direction (the X-axis direction in the present embodiment), and a projection portion 521 protruding upward from the proximal end portion 522. The upper surface of the projection portion 521 is exposed to the outside of the housing 510 and receives an operation by the operator. A plurality of teeth 523 aligned in the X-axis direction are formed on the lower surface of the proximal end portion 522. Further, wing portions 524 that protrude laterally (in the Z-axis positive direction and the Z-axis negative direction) are formed on both side surfaces of the proximal end portion 522. In the present embodiment, three wing portions 524 aligned in the X-axis direction are formed on both side surfaces of the proximal end portion 522. Each wing portion 524 has a substantially flat plate shape that is substantially perpendicular to the vertical direction (the Y-axis direction). In the present embodiment, the widths (sizes in the X-axis direction) of the three wing portions 524 formed on each side surface of the proximal end portion 522 are different from each other.

The rack case 530 is a box that houses the wire rack member 520. The rack case 530 is housed in the housing 510 so as to be slidable in a predetermined direction (the X-axis direction in the present embodiment). A range in which the rack case 530 can slide inside the housing 510 is set from a position where the rack case 530 and the housing 510 abut on each other on one side in the sliding direction to a position where the rack case 530 and the housing 510 abut on each other on the other side in the sliding direction. Thus, the rack case 530 (and the wire rack member 520 housed in the rack case 530) can perform a rectilinear motion in a predetermined direction with respect to the housing 510 for a predetermined distance. Further, a second biasing member 518 (see FIG. 10) such as a spring is disposed between the proximal end portion of the rack case 530 and the housing 510, and the rack case 530 is biased by the second biasing member 518 toward the start point of the rectilinear motion (i.e., toward the proximal end side in the present embodiment).

Further, a first biasing member 519 (see FIG. 14) such as a spring is disposed between the rack case 530 and the wire rack member 520, and the wire rack member 520 is biased upward (i.e., in a direction away from the driven gear 570 described below) with respect to the rack case 530 by the first biasing member 519. When the wire rack member 520 is not operated, substantially the entire projection portion 521 of the wire rack member 520 protrudes outside the housing 510 through an opening provided in the housing 510 due to the biasing force of the first biasing member 519. Note that, in the present embodiment, one first biasing member 519 is provided at each position of the distal end portion and the proximal end portion of the wire rack member 520.

Further, a partition wall 516 (see FIG. 12) extending in the X-axis direction is formed in the housing 510 at a position facing the wing portions 524 of the wire rack member 520 in the vertical direction (the Y-axis direction). The partition wall 516 has a substantially flat plate shape that is substantially perpendicular to the vertical direction. When the wire rack member 520 is biased by the first biasing member 519 and positioned upward, the wing portions 524 of the wire rack member 520 are positioned above the partition wall 516. Further, a plurality of cutouts 517 are formed in the partition wall 516. The positions and sizes of the plurality of cutouts 517 are set such that, when the position of the wire rack member 520 in the X-axis direction is at the start point and the end point of the rectilinear motion described above, each wing portion 524 of the wire rack member 520 can pass through the corresponding cutout 517 in the partition wall 516 and move in the vertical direction, and when the wire rack member 520 is positioned at any other position, each wing portion 524 of the wire rack member 520 is obstructed by the partition wall 516 and cannot move in the vertical direction. The partition wall 516 is an example of a movement restricting portion in the claims. Note that, in the rack case 530, openings 533 through which the wing portions 524 of the wire rack member 520 can pass are formed so as not to hinder the vertical movement of the wire rack member 520. Further, a groove 535 extending in the X-axis direction is formed in the side surface of the rack case 530, and the partition wall 516 is accommodated in the groove 535. Thus, the movement of the rack case 530 along the X-axis direction is not restricted by the existence of the partition wall 516. In other words, the rack case 530 can slide along the partition wall 516 in the predetermined direction (the X-axis direction).

The roller pair 542 is composed of two rollers that rotate about a rotation axis in a predetermined direction (the Z-axis direction in the present embodiment). The roller pair 542 is disposed so as to hold the guide groove 512 therebetween at a position on the distal end side of the bobbin 554 in the housing 510. The roller pair 542 holds therebetween the wire 502 which is extending from the position of the bobbin 554 and inserted through the guide groove 512. The roller pair 542 advances and retreats the wire 502 by rotating while holding the wire 502 therebetween.

The wire transmission mechanism 540 includes the driven gear 570 that rotates about a rotation axis in a predetermined direction (the Z-axis direction in the present embodiment). The driven gear 570 is disposed in the housing 510 at a position where it meshes with the teeth 523 of the wire rack member 520 when the wire rack member 520 moves downward. When the wire rack member 520 performs the rectilinear motion described above in a state where the driven gear 570 and the teeth 523 of the wire rack member 520 are meshed, the driven gear 570 rotates. Further, the wire transmission mechanism 540 includes a plurality of (five in the present embodiment) gears that mesh with each other. The wire transmission mechanism 540 converts the rotational force of the driven gear 570 associated with the rectilinear motion of the wire rack member 520 into a force for rotating the roller pair 542, thereby transmitting the force to the roller pair 542. Thus, when the wire rack member 520 performs the rectilinear motion while meshing with the driven gear 570, the roller pair 542 rotates, and as a result the wire 502 held between the roller pair 542 advances and retreats. Note that, in the present embodiment, the reduction ratio by the wire transmission mechanism 540 is set to a value such that the amount of movement of the wire 502 is several times the amount of movement of the wire rack member 520 in one rectilinear motion (e.g., about 8:1 to 16:1). That is, it is set to satisfy the following: the movement amount of the wire rack member 520 in one rectilinear motion/the movement amount of the wire 502=about 1/8 to 1/16.

The counter portion 552 is a substantially disk-shaped member and is housed in the housing 510 so as to be rotatable about a rotation axis in a predetermined direction (the Z-axis direction in the present embodiment). Numbers from 1 to 9 are written on the side surface of the counter portion 552. As shown in FIG. 1, a window 514 is formed at a position corresponding to the counter portion 552 in the housing 510, and one of the numbers formed on the side surface of the counter portion 552 is exposed through the window 514.

The counter transmission mechanism 550 includes the plurality of (five in the present embodiment) gears that mesh with each other, and converts the rotational force of the driven gear 570 associated with the rectilinear motion of the wire rack member 520 into a force for rotating the counter portion 552, thereby transmitting the force to the counter portion 552. Thus, when the wire rack member 520 performs the rectilinear motion while meshing with the driven gear 570, the counter portion 552 rotates. The reduction ratio by the counter transmission mechanism 550 is set to a value such that the number of the counter portion 552 exposed through the window 514 of the housing 510 increases by one per rectilinear motion of the wire rack member 520 (e.g., about 1:6 to 1:14). Thus, the counter portion 552 can display the number of times the wire rack member 520 has performed the rectilinear motion. Note that, in the present embodiment, one driven gear 570 is shared by the wire transmission mechanism 540 and the counter transmission mechanism 550.

A-6. Operation of plunger 50:
FIG. 15 is an explanatory diagram showing the operation of the plunger 50. As described above, the wire rack member 520 is biased upward (i.e., in the direction away from the driven gear 570) with respect to the rack case 530 by the first biasing member 519. Further, the rack case 530 housing the wire rack member 520 is biased by the second biasing member 518 toward the start point (i.e., toward the proximal end side) of the rectilinear motion of the rack case 530 and the wire rack member 520. Thus, in the initial state in which the wire rack member 520 is not operated by the operator, the wire rack member 520 is separated upward from the driven gear 570 and positioned at the start point of the rectilinear motion. In this state, even if the operator slides the wire rack member 520 to the distal end side by moving the thumb placed on the upper surface of the projection portion 521 of the wire rack member 520 to the distal end side, the driven gear 570 does not rotate as the teeth 523 of the wire rack member 520 are not engaged with the driven gear 570. Thus, the wire 502 is not sent out by the rotation of the roller pair 542, and the display of the number of times the wire rack member 520 has performed the rectilinear motion is not updated by the rotation of the counter portion 552.

As described above, the positions and sizes of the plurality of cutouts 517 formed in the partition wall 516 in the housing 510 are set such that, when the position of the wire rack member 520 is at the start point and the end point of the rectilinear motion, each wing portion 524 of the wire rack member 520 can pass through the corresponding cutout 517 in the partition wall 516 and move in the vertical direction. Thus, in the initial state, when the operator puts the thumb on the upper surface of the projection portion 521 of the wire rack member 520 and pushes the wire rack member 520 downward, as shown in FIG. 15, each wing portion 524 of the wire rack member 520 passes through the corresponding cutout 517 in the partition wall 516, and the wire rack member 520 is moved downward to a position where the teeth 523 of the wire rack member 520 mesh with the driven gear 570. While the state in which the wire rack member 520 is pushed downward is maintained in the manner, the operator moves the thumb placed on the upper surface of the projection portion 521 in the wire rack member 520 to the distal end side, thereby sliding the wire rack member 520 to the distal end side. As a result, the driven gear 570 rotates in accordance with the rectilinear motion of the wire rack member 520. When the driven gear 570 rotates, the rotation is converted into a force for rotating the roller pair 542 by the wire transmission mechanism 540, thereby transmitting the force to the roller pair 542. Thus, the roller pair 542 rotates, and the wire 502 held between the roller pair 542 is sent forward. Since the wire 502 is inserted into the drug solution storage lumen 40L of the needle catheter 40, when the wire 502 advances, as shown in the lower part of FIG. 15, a drug solution DS filled in the drug solution storage lumen 40L is pushed out by the wire 502, and the drug solution DS in an amount corresponding to the volume of the advanced wire 502 is ejected from the distal end of the needle portion 42. Note that, since the forward distance of the wire 502 associated with one rectilinear motion of the wire rack member 520 is predetermined by the reduction ratio of the wire transmission mechanism 540, the ejection amount of the drug solution DS associated with one rectilinear motion of the wire rack member 520 is also predetermined. The wire rack member 520, the driven gear 570, the wire transmission mechanism 540, and the roller pair 542 are examples of a wire operation section in the claims.

Note that, as described above, the positions and sizes of the plurality of cutouts 517 formed in the partition wall 516 in the housing 510 are set such that, when the wire rack member 520 is at a position other than the start point and the end point of the rectilinear motion, the wing portions 524 of the wire rack member 520 are obstructed by the partition wall 516 and cannot move in the vertical direction. Thus, the teeth 523 of the wire rack member 520 are kept in mesh with the driven gear 570 until the wire rack member 520 reaches the end point of the rectilinear motion. Further, when the wire rack member 520 reaches the end point of the rectilinear motion and the operator stops operating the wire rack member 520, the wire rack member 520 is moved upward by the biasing force of the first biasing member 519, and the engagement between the teeth 523 of the wire rack member 520 and the driven gear 570 is released. Further, the wire rack member 520 is returned to the start point of the rectilinear motion by the biasing force of the second biasing member 518.

Further, when the wire rack member 520 performs the rectilinear motion from the start point to the end point while engaging with the driven gear 570, the driven gear 570 rotates, and the rotation is converted by the counter transmission mechanism 550 into the force for rotating the counter portion 552, thereby transmitting the force to the counter portion 552. Thus, the counter portion 552 rotates, and the display of the number of times the wire rack member 520 has performed the rectilinear motion is updated to a value increased by one.

A-7. Method of using the drug solution injection device 10:
A method of using the drug solution injection device 10 is, for example, as follows. The method of using the drug solution injection device 10 by two operators will be described below. First, a first operator inserts the sheath 21 housing the needle catheter 40 into an endoscope and moves the distal end of the sheath 21 to the vicinity of a target position (position where the drug solution is administered) in the body of the patient. Note that this movement is performed in a state in which the needle portion 42 of the needle catheter 40 does not protrude from the distal end of the sheath 21 and is housed in the sheath 21. After that, a second operator supplies the drug solution into the drug solution storage lumen 40L through the connector 45 of the needle catheter 40.

Next, the first operator operates the operating part 23 of the operation catheter 20 to perform puncture with the needle portion 42 at the target position in the patient's body. More specifically, the first operator performs an operation of pushing the first dial 248 that constitutes the first operation input section 240 toward the distal end side, thereby causing the needle portion 42 of the needle catheter 40 to protrude from the distal end of the sheath 21 (see FIG. 7). Next, the first operator performs the rotary operation of the second dial 268 that constitutes the second operation input section 260 to bend the distal end portion of the sheath 21 and the distal end portion of the needle catheter 40 housed in the sheath 21 (see FIG. 8), or performs the rotary operation of the first dial 248 that constitutes the first operation input section 240 to rotate the sheath 21 and the needle catheter 40 housed in the sheath 21, thereby precisely setting the position and direction of the needle portion 42 to the position and direction corresponding to the desired puncture position. Thereafter, the first operator performs an operation of pushing the entire operating part 23 toward the distal end side, thereby performing puncture with the needle portion 42.

After that, the second operator operates the wire rack member 520 of the plunger 50 and causes the wire rack member 520 to perform the rectilinear motion, thereby advancing the wire 502 in the drug solution storage lumen 40L of the needle catheter 40 and ejecting the drug solution DS from the distal end of the needle portion 42 (see FIG. 15). As described above, since the ejection amount of the drug solution DS associated with one rectilinear motion of the wire rack member 520 is predetermined, the second operator repeats the operation of the rectilinear motion of the wire rack member 520 until the amount of the drug solution DS to be administered to the current puncture position is ejected. During this operation, each time the wire rack member 520 performs the rectilinear motion, the number of the counter portion 552 exposed through the window 514 of the housing 510 is increased by one. Thus, the second operator can accurately recognize the number of times the wire rack member 520 has performed the rectilinear motion, that is, the amount of the drug solution DS that has already been administered.

When the administration of a predetermined amount of the drug solution DS to the puncture position is completed, the first operator performs an operation of pulling back the entire operating part 23 to the proximal end side, thereby pulling out the needle portion 42 from the punctured position. After that, in the same manner, the first operator adjusts the position and direction of the needle portion 42 to another puncture position and punctures the needle portion 42 at that position. Then, the second operator administers the drug solution DS.

When such operations are repeated and the administration of the predetermined amount of the drug solution DS to all puncture positions is completed, the first operator performs an operation of pulling back the first dial 248 constituting the first operation input section 240 to the proximal end side, thereby housing the needle portion 42 of the needle catheter 40 inside the sheath 21. In this state, the first operator pulls the operating part 23 to the proximal end side to pull out the sheath 21 and the needle catheter 40 from the patient's body.

A-8. Effects of first embodiment:
As described above, the drug solution injection device 10 of the present embodiment includes the operation catheter 20. The operation catheter 20 includes the sheath 21 and the operating part 23 that is attached to the proximal end portion of the sheath 21 and operates the sheath 21. The operating part 23 includes the rotary operation section (the first operation input section 240 and the rotary shaft 220) that rotates the sheath 21 about the central axis of the sheath 21, and the bending operation section (the second operation input section 260, the transmission mechanism 270, the sliding member 230, and the operation wire 226) that bends the distal end portion of the sheath 21.

In this manner, in the drug solution injection device 10 of the present embodiment, the operating part 23 includes the rotary operation section that rotates the sheath 21 and the bending operation section that bends the distal end portion of the sheath 21, making it possible to freely change the position and direction of the distal end portion of the sheath 21. Thus, according to the drug solution injection device 10 of the present embodiment, the operability of the drug solution injection device 10 can be improved.

Further, in the drug solution injection device 10 of the present embodiment, the rotary operation section that rotates the sheath 21 includes the first operation input section 240 and the rotary shaft 220. The first operation input section 240 rotates about the central axis AX in accordance with the operator's operation, thereby rotating the sheath 21 about the central axis AX, and the first operation input section 240 is displaceable relative to the sheath 21 in the central axis AX direction. The rotary shaft 220 is attached to the first operation input section 240 so as to rotate as the first operation input section 240 rotates, and the rotary shaft 220 is attached to the proximal end portion of the sheath 21. Thus, according to the drug solution injection device 10 of the present embodiment, the rotation of the sheath 21 (and the catheter shaft 43 of the needle catheter 40 housed in the sheath 21, the same applies hereafter) can be achieved by an operator's intuitive and simple operation, thereby making it possible to improve the operability of the drug solution injection device 10.

Further, in the drug solution injection device 10 of the present embodiment, the bending operation section that bends the distal end portion of the sheath 21 includes the sliding member 230 that is slidably attached to the rotary shaft 220 in the central axis AX direction, the operation wire 226 connecting the sliding member 230 and the distal end portion of the sheath 21, the second operation input section 260 that rotates in accordance with the operator's operation, and the transmission mechanism 270 that converts the rotational force applied to the second operation input section 260 into the force for sliding the sliding member 230, thereby transmitting the force to the sliding member 230. Thus, according to the drug solution injection device 10 of the present embodiment, bending of the distal end portion of the sheath 21 can be achieved by an operator's simple operation, making it possible to improve the operability of the drug solution injection device 10.

Further, in the drug solution injection device 10 of the present embodiment, the sliding member 230 is configured to maintain a state of receiving the force for sliding the sliding member 230 from the transmission mechanism 270 even when the rotary shaft 220 rotates about the central axis AX. Thus, according to the drug solution injection device 10 of the present embodiment, the bending operation of the distal end portion of the sheath 21 can be performed regardless of the rotation angle of the sheath 21, making it possible to effectively improve the operability of the drug solution injection device 10.

Further, in the drug solution injection device 10 of the present embodiment, the sliding member 230 includes the core member 232 attached to the rotary shaft 220 so as to rotate as the rotary shaft 220 rotates about the central axis AX, and the rack member 231 that is attached to the core member 232 so as to be rotatable relative to the core member 232 about the central axis AX and to slide in accordance with the sliding of the core member 232 in the central axis AX direction, and has the plurality of teeth 234 that mesh with the plurality of teeth of the transmission mechanism 270. Thus, according to the drug solution injection device 10 of the present embodiment, it is possible to reduce the number of places where the teeth 234 are formed in the rack member 231, thereby achieving simplification and efficiency in processing.

Further, the drug solution injection device 10 of the present embodiment further includes the needle catheter 40. The needle catheter 40 is slidably inserted into the sheath 21 via the operating part 23, and includes the catheter shaft 43 having the drug solution storage lumen 40L for storing the drug solution. In this manner, in the drug solution injection device 10 of the present embodiment, the sheath 21 housing the catheter shaft 43 of the needle catheter 40 can be rotated by the rotary operation section of the operating part 23, and the distal end portion of the sheath 21 housing the catheter shaft 43 of the needle catheter 40 can be bent by the bending operation section of the operating part 23, making it possible to freely change the position and direction of the distal end portion of the catheter shaft 43 of the needle catheter 40 having the drug solution storage lumen 40L for storing the drug solution. Thus, according to the drug solution injection device 10 of the present embodiment, it is possible to improve not only the operability of the drug solution injection device 10 but also the accuracy of the drug solution injection position.

Further, in the drug solution injection device 10 of the present embodiment, the rotary operation section that rotates the sheath 21 includes the first operation input section 240 and the rotary shaft 220. The first operation input section 240 rotates about the central axis AX in accordance with the operator's operation, thereby rotating the sheath 21 about the central axis AX, and the first operation input section 240 is displaceable relative to the sheath 21 in the central axis AX direction. The rotary shaft 220 is attached to the first operation input section 240 so as to rotate as the first operation input section 240 rotates, and the rotary shaft 220 is attached to the proximal end portion of the sheath 21. The first operation input section 240 is fixed to the needle catheter 40 via the relay catheter 30. Further, the needle catheter 40 is configured such that the distal end of the needle catheter 40 is housed in the sheath 21 when the first operation input section 240 is at the first position, and the distal end of the needle catheter 40 protrudes from the distal end of the sheath 21 when the first operation input section 240 is at the second position on the distal end side from the first position. According to the drug solution injection device 10 of the present embodiment, the sheath 21 and the catheter shaft 43 can be moved to the drug solution injection position while the distal end of the catheter shaft 43 of the needle catheter 40 is housed in the sheath 21, making it possible to prevent the distal end of the catheter shaft 43 from damaging the inside of the patient's body. Further, according to the drug solution injection device 10 of the present embodiment, the distal end of the catheter shaft 43 can be protruded from the distal end of the sheath 21 at the drug solution injection position by an intuitive and simple operation, making it possible to improve the operability of the drug solution injection device 10.

Further, in the drug solution injection device 10 of the present embodiment, the distal end of the catheter shaft 43 of the needle catheter 40 is provided with the needle portion 42. The first operation input section 240 of the operating part 23 is attached to the rotary shaft 220 so as to rotate with the rotation of the rotary shaft 220 about the central axis AX and to be displaceable relative to the rotary shaft 220 in the central axis AX direction. The needle catheter 40 is configured such that the needle portion 42 is housed in the sheath 21 when the first operation input section 240 is at the first position, and the needle portion 42 protrudes from the distal end of the sheath 21 when the first operation input section 240 is at the second position on the distal end side from the first position. Thus, according to the drug solution injection device 10 of the present embodiment, the sheath 21 and the needle catheter 40 housed in the sheath 21 can be moved to the puncture position while the needle portion 42 of the needle catheter 40 is housed in the sheath 21, making it possible to prevent the needle portion 42 from damaging the inside of the patient's body. Further, according to the drug solution injection device 10 of the present embodiment, at the puncture position, the needle portion 42 can be protruded from the distal end of the sheath 21 by an intuitive and simple operation, making it possible to improve the operability of the drug solution injection device 10.

Further, the drug solution injection device 10 of the present embodiment further includes the wire 502 slidably inserted into the drug solution storage lumen 40L of the catheter shaft 43 of the needle catheter 40, and the plunger 50 separate from the operation catheter 20. The plunger 50 includes the bobbin 554 that houses the wire 502, and the wire operation section (the wire rack member 520, the driven gear 570, the wire transmission mechanism 540, and the roller pair 542) that advances and retracts the wire 502 in the drug solution storage lumen 40L by sending out the wire 502 from the bobbin 554 or pulling the wire 502 back to the bobbin 554. Thus, according to the drug solution injection device 10 of the present embodiment, the operation of the sheath 21 (and the needle catheter 40 housed in the sheath 21) by the operating part 23 of the operation catheter 20 and the drug solution ejection operation by the plunger 50 can be performed by different operators. As a result, the accuracy of both operations can be improved.

Further, in the drug solution injection device 10 of the present embodiment, the wire operation section includes the wire rack member 520 which has the plurality of teeth 523 and performs the rectilinear motion of a predetermined length, the roller pair 542 that holds the wire 502 therebetween and moves the wire 502 forward and backward by rotating, and the wire transmission mechanism 540 that includes the plurality of gears including the driven gear 570 having the plurality of teeth meshing with the plurality of teeth 523 of the wire rack member 520, and converts the rectilinear motion of the wire rack member 520 into the rotary motion of the roller pair 542, thereby transmitting the motion to the roller pair 542. Thus, according to the drug solution injection device 10 of the present embodiment, the wire rack member 520 is operated to perform the rectilinear motion of a predetermined length, thereby causing the wire 502 to advance by a certain amount in the drug solution storage lumen 40L. As a result, a predetermined amount of the drug solution can be ejected from the distal end of the needle catheter 40, making it possible to accurately control the ejection amount of the drug solution.

Further, in the drug solution injection device 10 of the present embodiment, the plunger 50 further includes the first biasing member 519 for biasing the wire rack member 520 to be away from the driven gear 570. Thus, according to drug solution injection device 10 of the present embodiment, when the wire rack member 520 is unintentionally caused to perform the rectilinear motion, the driven gear 570 does not rotate, making it possible to prevent the drug solution from being ejected due to an erroneous operation.

Further, in the drug solution injection device 10 of the present embodiment, the plunger 50 further includes the partition wall 516 which allows the wire rack member 520 to move in a direction toward the driven gear 570 and in a direction away from the driven gear 570 when the wire rack member 520 is at the start point and the end point of the rectilinear motion, and restricts the wire rack member 520 from moving in the direction toward the driven gear 570 or in the direction away from the driven gear 570 when the wire rack member 520 is at any other position. Thus, according to the drug solution injection device 10 of the present embodiment, the wire rack member 520 can be prevented from being unintentionally separated from the driven gear 570 while the wire rack member 520 is engaged with the driven gear 570 and performs the rectilinear motion. This can prevent that the ejection amount of the drug solution associated with one rectilinear motion of the wire rack member 520 becomes less than the predetermined amount. Further, when the wire rack member 520 that is not engaged with the driven gear 570 is at a position other than the start point and the end point of the rectilinear motion, it is possible to prevent unintentional engagement of the wire rack member 520 with the driven gear 570 and unintended ejection of the drug solution, making it possible to effectively improve the accuracy of the ejection amount of the drug solution.

Further, in the drug solution injection device 10 of the present embodiment, the plunger 50 further includes the second biasing member 518 for biasing the wire rack member 520 toward the start point of the rectilinear motion. Thus, according to the drug solution injection device 10 of the present embodiment, there is no need for the operator to perform the operation of moving the wire rack member 520 to the position of the start point of the rectilinear motion, making it possible to effectively improve the operability of the drug solution injection device 10.

Further, in the drug solution injection device 10 of the present embodiment, the plunger 50 further includes the counter portion 552 that displays the number of times the wire rack member 520 has performed the rectilinear motion by rotating, and the counter transmission mechanism 550 that includes the plurality of gears including the driven gear 570 having the plurality of teeth that mesh with the plurality of teeth 523 of the wire rack member 520, and converts the rectilinear motion of the wire rack member 520 into the rotary motion of the counter portion 552, thereby transmitting the motion to the counter portion 552. Thus, according to the drug solution injection device 10 of the present embodiment, as the wire rack member 520 performs the rectilinear motion, the counter portion 552 rotates and updates the display of the number of times the rectilinear motion has been performed, making it possible to accurately recognize the number of times the wire rack member 520 has performed the rectilinear motion, that is, the amount of the drug solution that has already been administered.

Further, in the drug solution injection device 10 of the present embodiment, the wire transmission mechanism 540 and the counter transmission mechanism 550 share the driven gear 570. Thus, according to the drug solution injection device 10 of the present embodiment, while achieving the simplification of the configuration of the plunger 50, it is possible to achieve the ejection of the drug solution and the update of the display of the number of times associated with the rectilinear motion of the wire rack member 520.

B. Second embodiment:

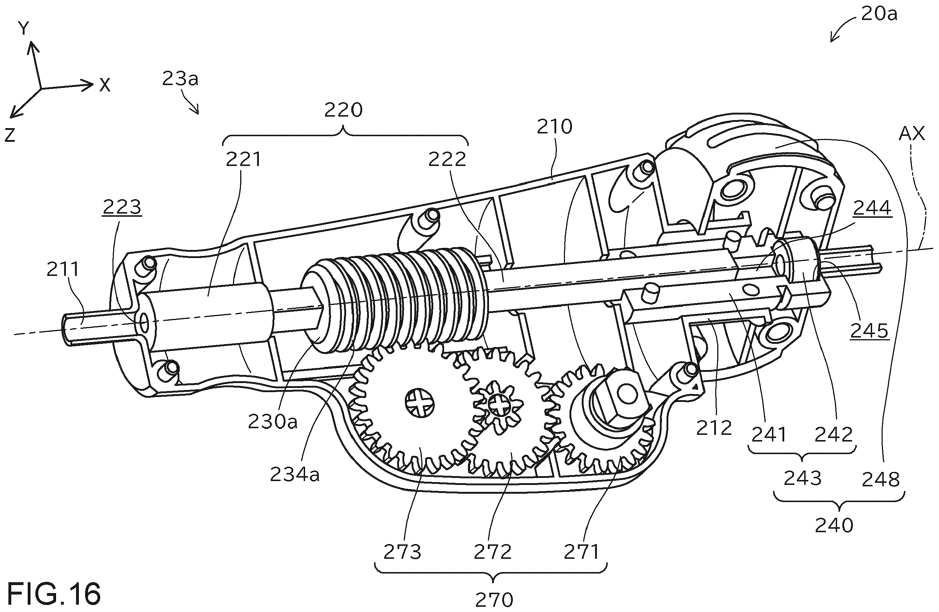

FIG. 16 is an explanatory diagram schematically showing configurations of an operation catheter 20a in a second embodiment. In the following, among the configurations of the operation catheter 20a in the second embodiment, the same configurations as those of the operation catheter 20 in the first embodiment described above are denoted by the same reference signs, and description thereof is omitted as appropriate.

In the operation catheter 20a in the second embodiment, a sliding member 230a of an operating part 23a is configured by a single rack member. That is, the sliding member 230a is a substantially cylindrical rack member having a plurality of teeth 234a that engage with the plurality of teeth of the transmission mechanism 270 formed on its outer peripheral surface. The sliding member 230a is attached to the rotary shaft 220 so as to rotate as the rotary shaft 220 rotates about the central axis AX. As described above, in the second embodiment, the sliding member 230a is the substantially cylindrical rack member. Thus, the sliding member 230a maintains a state of receiving the force for sliding the sliding member 230a from the transmission mechanism 270 even when the rotary shaft 220 rotates about the central axis AX.

Thus, in the second embodiment, as in the first embodiment, the sliding member 230a of the operating part 23a of the operation catheter 20a is configured to maintain the state of receiving the force for sliding the sliding member 230a from the transmission mechanism 270 even if the rotary shaft 220 rotates about the central axis AX, making it possible to perform the bending operation of the distal end portion of the sheath 21 regardless of the rotation angle of the sheath 21. This can effectively improve the operability of the drug solution injection device 10.

Further, in the second embodiment, the sliding member 230a is the substantially cylindrical rack member having the plurality of teeth 234a that mesh with the plurality of teeth of the transmission mechanism 270 formed on its outer peripheral surface, and the sliding member 230a is attached to the rotary shaft 220 so as to rotate as the rotary shaft 220 rotates about the central axis AX. Thus, according to the second embodiment, it is possible to reduce the number of parts of the operating part 23a, thereby achieving simplification and efficiency in production.

C. Modifications:
The technology disclosed in the present specification is not limited to the above-mentioned embodiments, and can be modified in various forms without departing from the scope of the present specification. For example, the following modifications can be made.

The configurations of the drug solution injection device 10 in the above embodiments are merely examples, and various modifications can be made. For example, in the above embodiments, the drug solution injection device 10 includes the operation catheter 20, the relay catheter 30, the needle catheter 40, the plunger 50, and the wire 502. However, at least one of them can be omitted or changed to other configurations. More specifically, for example, the relay catheter 30 may be omitted, and instead of the plunger 50 and the wire 502 for performing the ejection operation of the drug solution, other configurations may be used to achieve the ejection of the drug solution. Further, the plunger 50 and the wire 502 do not need to be used in combination with the operation catheter 20 or the relay catheter 30, and the drug solution injection device may be configured by the needle catheter 40, the plunger 50, and the wire 502.

In the above embodiments, the configuration for rotating the sheath 21 in the operating part 23 of the operation catheter 20, the configuration for bending the distal end portion of the sheath 21, and the configuration for protruding the needle portion 42 of the needle catheter 40 from the distal end of the sheath 21 are merely examples, and other configurations can be adopted.

In the above embodiments, the configuration for advancing and retracting the wire 502 in the plunger 50 and the configuration for displaying the number of times the wire rack member 520 has performed the rectilinear motion are merely examples, and other configurations can be adopted. Further, in the plunger 50, the first biasing member 519 and/or the second biasing member 518 may be omitted. Further, in the plunger 50, the configuration for displaying the number of times the wire rack member 520 has performed the rectilinear motion may be omitted. Further, in the plunger 50, the wire transmission mechanism 540 and the counter transmission mechanism 550 may have different driven gears 570.

The methods of using the drug solution injection device 10 in the above embodiments are merely an example, and the drug solution injection device 10 may be used with other methods.

In the above embodiments, the drug solution injection device 10 including the operation catheter 20 and the needle catheter 40 has been described. However, the technology disclosed in the present specification is applicable to not only such a drug solution injection device 10 but also catheter devices in general. For example, the technology disclosed in the present specification can be implemented as a catheter device that includes the operation catheter 20 but not the needle catheter 40. In such a catheter device, by adopting a configuration in which the operation catheter 20 includes the sheath 21 and the operating part 23 that is attached to the proximal end portion of the sheath 21 and operates the sheath 21, and the operating part 23 include the rotary operation section that rotates the sheath 21 about the central axis of the sheath 21 and the bending operation section that bends the distal end portion of the sheath 21, it is possible to freely change the position and direction of the distal end portion of the sheath 21 by the rotary operation section and the bending operation section, thereby improving the operability of the catheter device.

### DESCRIPTION OF REFERENCE NUMERALS

- 10:: Drug solution injection device

- 20:: Operation catheter
- 21:: Sheath
- 22:: Lumen
- 23:: Operating part
- 30:: Relay catheter
- 40:: Needle catheter
- 40L:: Drug solution storage lumen
- 41:: Shaft main body portion
- 42:: Needle portion
- 43:: Catheter shaft
- 45:: Connector
- 50:: Plunger
- 210:: Housing
- 211:: Tip side guide portion
- 212:: Base side guide portion
- 220:: Rotary shaft
- 221:: Tip portion
- 222:: Base portion
- 223:: Lumen
- 226:: Operation wire
- 230:: Sliding member
- 231:: Rack member
- 232:: Core member
- 234:: Tooth
- 235:: Tip portion
- 236:: Intermediate portion
- 237:: Base portion
- 240:: First operation input section
- 241:: Connection main body portion
- 242:: Annular member
- 243:: Connecting member
- 244:: Lumen
- 245:: Enlarged diameter portion
- 248:: First dial
- 260:: Second operation input section
- 268:: Second dial
- 269:: Stopper
- 270:: Transmission mechanism
- 271, 272, 273:: Gear
- 502:: Wire
- 510:: Housing
- 511:: Tip surface
- 512:: Guide groove
- 514:: Window
- 516:: Partition wall
- 517:: Cutout
- 518:: Second biasing member
- 519:: First biasing member
- 520:: Wire rack member
- 521:: Projection portion
- 522:: Base portion
- 523:: Tooth
- 524:: Wing portion
- 530:: Rack case
- 533:: Opening
- 535:: Groove
- 540:: Wire transmission mechanism
- 542:: Roller pair
- 550:: Counter transmission mechanism
- 552:: Counter portion
- 554:: Bobbin
- 570:: Driven gear
- AX:: Central axis
- DS:: Drug solution

## Claims

1. A catheter device comprising a first catheter including a first catheter shaft and an operating part that is attached to a proximal end portion of the first catheter shaft and operates the first catheter shaft, wherein the operating part includes:
a rotary operation section that rotates the first catheter shaft about a central axis of the first catheter shaft; and
a bending operation section that bends a distal end portion of the first catheter shaft.

2. The catheter device according to claim 1, wherein the rotary operation section includes:
a first operation input section that rotates the first catheter shaft about the central axis by rotating about the central axis in accordance with an operation by an operator, the first operation input section being displaceable relative to the first catheter shaft in a direction of the central axis; and
a rotary shaft that is attached to the first operation input section so as to rotate as the first operation input section rotates, the rotary shaft being attached to the proximal end portion of the first catheter shaft.

3. The catheter device according to claim 2, wherein the bending operation section includes:
a sliding member attached to the rotary shaft so as to be slidable in the central axis direction;
an operation wire that connects the sliding member and the distal end portion of the first catheter shaft;
a second operation input section that rotates in accordance with the operation by the operator: and
a transmission mechanism that converts a rotational force of the second operation input section into a force for sliding the sliding member, thereby transmitting the force to the sliding member.

4. The catheter device according to claim 3, wherein the sliding member is configured to maintain a state of receiving the force for sliding the sliding member from the transmission mechanism when the rotary shaft rotates about the central axis.

5. The catheter device according to claim 4, wherein the sliding member includes:
a core member attached to the rotary shaft so as to rotate as the rotary shaft rotates about the central axis; and
a rack member attached to the core member so as to be rotatable relative to the core member about the central axis and to slide as the core member slides in the central axis direction, the rack member having a plurality of teeth that mesh with a plurality of teeth of the transmission mechanism.

6. The catheter device according to claim 4, wherein the sliding member is a substantially cylindrical rack member having a plurality of teeth meshing with a plurality of teeth of the transmission mechanism formed on an outer peripheral surface of the rack member, the sliding member being attached to the rotary shaft so as to rotate as the rotary shaft rotates about the central axis.

7. A drug solution injection device comprising:
the catheter device according to any one of claims 1 to 6; and
a second catheter including a second catheter shaft that is slidably inserted into the first catheter shaft via the operating part and has a drug solution storage lumen for storing a drug solution.

8. The drug solution injection device according to claim 7, wherein the rotary operation section includes:
a first operation input section that rotates the first catheter shaft about the central axis by rotating about the central axis in accordance with an operation by an operator, the first operation input section being displaceable relative to the first catheter shaft in the central axis direction; and
a rotary shaft that is attached to the first operation input section so as to rotate as the first operation input section rotates, the rotary shaft being attached to the proximal end portion of the first catheter shaft, wherein:
the first operation input section is fixed to the second catheter directly or via another member; and
the second catheter is configured such that a distal end of the second catheter is housed in the first catheter shaft when the first operation input section is at a first position, and the distal end of the second catheter protrudes from a distal end of the first catheter shaft when the first operation input section is at a second position on a distal end side from the first position.

9. The drug solution injection device according to claim 8, wherein:
a needle portion is provided at a distal end of the second catheter shaft;
the first operation input section of the rotary operation section is attached to the rotary shaft so as to rotate as the rotary shaft rotates about the central axis and to be displaceable relative to the rotary shaft in the central axis direction; and
the second catheter is configured such that the needle portion is housed in the first catheter shaft when the first operation input section is at the first position, and the needle portion protrudes from the distal end of the first catheter shaft when the first operation input section is at the second position.

10. The drug solution injection device according to any one of claims 7 to 9, further comprising:
a wire slidably inserted into the drug solution storage lumen of the second catheter shaft; and
a plunger separate from the first catheter, wherein the plunger includes:
a wire housing portion for housing the wire; and
a wire operation section that advances and retracts the wire in the drug solution storage lumen by sending out the wire from the wire housing portion or pulling the wire back into the wire housing portion.

11. The drug solution injection device according to claim 10, wherein the wire operation section includes:
a wire rack member that has a plurality of teeth and performs a rectilinear motion for a predetermined length;
a roller pair that holds the wire between the roller pair and advances and retracts the wire by rotating; and
a wire transmission mechanism that includes a plurality of gears including a driven gear having a plurality of teeth meshing with the plurality of teeth of the wire rack member, the wire transmission mechanism converting the rectilinear motion of the wire rack member into a rotary motion of the roller pair, thereby transmitting the motion to the roller pair.

12. The drug solution injection device according to claim 11, wherein the plunger further includes a first biasing member that biases the wire rack member to be away from the driven gear.

13. The drug solution injection device according to claim 12, wherein the plunger further includes a movement restricting portion that allows the wire rack member to move in a direction toward the driven gear and in a direction away from the driven gear when the wire rack member is at a start point and an end point of the rectilinear motion, and restricts the wire rack member from moving in the direction toward the driven gear or in the direction away from the driven gear when the wire rack member is at any other position.

14. The drug solution injection device according to claim 12 or 13, wherein the plunger further includes a second biasing member that biases the wire rack member toward the start point of the rectilinear motion.

15. The drug solution injection device according to any one of claims 11 to 14, wherein the plunger further includes:
a counter portion that rotates and displays the number of times the wire rack member has performed the rectilinear motion; and
a counter transmission mechanism that includes a plurality of gears including a driven gear having a plurality of teeth meshing with the plurality of teeth of the wire rack member, the counter transmission mechanism converting the rectilinear motion of the wire rack member into a rotary motion of the counter portion, thereby transmitting the motion to the counter portion.

16. The drug solution injection device according to claim 15, wherein the wire transmission mechanism and the counter transmission mechanism share the driven gear.
